(19) Europäisches Patentamt
European Patent Office
Office européen des brevets

(11) **EP 3 452 590 B1**

(12) **EUROPEAN PATENT SPECIFICATION**

(45) Date of publication and mention
of the grant of the patent:
**29.12.2021 Bulletin 2021/52**

(21) Application number: **17792345.5**

(22) Date of filing: **05.05.2017**

(51) Int Cl.:
*C12N 15/10* (2006.01)       *B03C 1/00* (2006.01)
*C07K 1/22* (2006.01)       *C12N 1/00* (2006.01)
*C12N 5/00* (2006.01)       *G01N 33/53* (2006.01)
*B03C 1/033* (2006.01)      *B03C 1/28* (2006.01)
*C12N 13/00* (2006.01)

(86) International application number:
**PCT/CA2017/050554**

(87) International publication number:
**WO 2017/190254 (09.11.2017 Gazette 2017/45)**

(54) **PLATE MAGNET**

PLATTENMAGNET

AIMANT SOUS FORME DE PLAQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.05.2016   US 201662332578 P**

(43) Date of publication of application:
**13.03.2019   Bulletin 2019/11**

(60) Divisional application:
**21209583.0**

(73) Proprietor: **Stemcell Technologies Inc.
Vancouver, British Columbia V6A 1B6 (CA)**

(72) Inventors:
• **JERVIS, Eric
Vancouver
British Columbia V6P 1S9 (CA)**
• **GLAWDEL, Tom
Toronto
Ontario M5V 4A2 (CA)**

(74) Representative: **HGF
HGF Limited
1 City Walk
Leeds LS11 9DX (GB)**

(56) References cited:
**WO-A1-99/42219       WO-A2-01/71034
WO-A2-2008/080047    WO-A2-2008/080047
US-A- 5 631 618**

• **LESTER C BARNSLEY ET AL: "Halbach arrays
consisting of cubic elements optimised for high
field gradients in magnetic drug targeting
applications", PHYSICS IN MEDICINE AND
BIOLOGY, INSTITUTE OF PHYSICS PUBLISHING,
BRISTOL GB, vol. 60, no. 21, 12 October 2015
(2015-10-12), pages 8303-8327, XP020290373,
ISSN: 0031-9155, DOI:
10.1088/0031-9155/60/21/8303 [retrieved on
2015-10-12]**
• **HOYOS, M ET AL.: 'The use of a linear Halbach
array combined with a step-SPLITT channel for
continuous sorting of magnetic species' J MAGN
MAGN MATER. vol. 323, no. 10, 01 May 2011,
pages 1384 - 1388, XP028182621**

## Description

FIELD

Embodiments described herein relate to magnets, and in particular magnetic arrays for magnetic separation methods and apparatus.

BACKGROUND

US Patent No. US 6,514,415B2 purports to describe a method and apparatus for magnetic separation of particles within a container. In one embodiment, a container contains a number of particles and a number of magnetically susceptible particles. A number of magnets are arranged in a plane and placed close to the container. The magnetic poles of the magnets are arranged in a pattern to apply magnetic fields oriented perpendicular to the plane on the container. The pole pattern provides a consistent separation across the container of the number of magnetically susceptible particles from the rest of the particles.

WO2008/080047 discloses arrangement of magnets that are used to separate magnetic particles from fluid samples.

(LESTER BARNSLEY ET AL: "Halbach arrays consisting of cubic elements optimized for high field gradients in magnetic drug targeting applications", PHYSICS IN MEDICINE AND BIOLOGY, vol. 60, no. 21, 12 October 2015, pages 8303-8327). Discloses the use of permanent magnets to deliver the maximum pull or push.

WO99/42219 discloses a magnetic system for continuous separation.

WO01/71034 discloses a method for manipulating biological entities using magnets.

SUMMARY

This summary is intended to introduce the reader to the more detailed description that follows and not to limit or define any claimed or as yet unclaimed subject matter. One or more inventions may reside in any combination or sub-combination of the elements or process steps disclosed in any part of this document including its claims and figures.

Embodiments described herein may provide a design for a plate magnet for use in a magnetic separation apparatus. In some cases, the magnet may be referred to as a universal plate magnet. Embodiments of the plate magnets described herein may be used for automation applications that can process a wide range of volumes in a reasonable time frame.

In a broad aspect, there is provided a magnetic separation apparatus as defined in claim 1 of the appended claims.

In some embodiments, the array surface is substantially planar.

In some embodiments, the first two-dimensional magnetic array is substantially rectangular.

In some embodiments, the plurality of magnets are enclosed by the housing. In some embodiments, the housing has an interior frame defining a two-dimensional frame array and the magnetic elements are positioned in the two-dimensional frame array to define the two-dimensional magnetic array.

In some embodiments, the housing defines a container receiving surface adjacent to the array surface.

In some embodiments, the magnetic separation apparatus further comprises a plurality of guide elements attached to the housing, the guide elements arranged to direct the one or more containers to a position adjacent the container receiving surface.

In some embodiments, the guide elements include at least one guide member extending from the housing in a direction perpendicular to the container receiving surface and away from the array surface.

In some embodiments, the housing is substantially rectangular; and the plurality of guide elements comprise a plurality of guide members extending from the housing, the plurality of guide members including at least one guide member positioned at each corner of the housing.

In some embodiments, the magnetic separation apparatus further comprises a second plurality of magnetic elements arranged to define a second two-dimensional magnetic array. In some embodiments, each of the first two-dimensional magnetic array and the second two-dimensional magnetic array may be supported by a housing.

In some embodiments, the first two-dimensional magnetic array and the second two-dimensional magnetic array are positioned in an at least partially facing arrangement with the array surface of the first two-dimensional magnetic array facing a second array surface of the second two-dimensional magnetic array; and the apparatus comprises a receiving area between the first two-dimensional magnetic array and the second two-dimensional magnetic array shaped to receive a container for a plurality of magnetically susceptible particles.

In some embodiments, the first two-dimensional magnetic array and the second two-dimensional magnetic array are substantially parallel.

**EP 3 452 590 B1**

In some embodiments, the first two-dimensional magnetic array and the second two-dimensional magnetic array are oriented to define a magnetic field strength minimum along a plane defined by the magnetic field strength minimum resulting from the magnetic field of the first two-dimensional magnetic array and the magnetic field of the second two-dimensional magnetic array.

In some embodiments, the first two-dimensional magnetic array and the second two-dimensional magnetic array are arranged back to back.

In some embodiments, the apparatus has a receiving area shaped to receive at least one container configured to contain a plurality of magnetically susceptible particles; and the at least one container comprises at least one of a tube, a vial, a Petri dish, a bottle, a bag, and a multi-well micro-plate.

In some embodiments, the magnetic elements comprise permanent magnets.

In a broad aspect, there is provided a method for magnetically separating a plurality of targets from a suspension in a container unit as defined in claim 11 of the appended claims.

In some embodiments, positioning the container unit in proximity to the array surface comprises guiding the container unit towards the array surface using a plurality of guide elements.

In some embodiments, the plurality of guide elements are arranged to correspond to a shape of the container unit.

In some embodiments, the container unit is one of a tube, a vial, a Petri dish, a bottle, a bag, and a multi-well micro-plate.

In some embodiments, the container unit comprises a plurality of container compartments, each container compartment shaped to receive an individual container for a cell suspension, and at least one of the container compartments comprises an in-use container with a corresponding cell suspension; and the method comprises repeating the linking and extracting steps for the corresponding cell suspension of each in-use container.

Also disclosed, but not claimed, is a magnetic separation apparatus for separating a plurality of magnetically linked targets in a container, the apparatus comprising: a plurality of magnetic elements fixedly arranged adjacent to each other forming a two-dimensional array having rows and columns, wherein respective magnetic fields of magnets of said plurality of magnetic elements in a first row of the array are arranged to form a first order Halbach array, and proceeding in a column direction of the array, respective magnetic fields of magnets of said plurality of magnets in each of a second and succeeding rows of the array are oriented in the same direction relative to respective magnetic fields of magnets of said plurality of magnets in any immediately preceding row, such that the plurality of magnets apply magnetic fields to the container to separate at least some of the plurality of magnetically linked targets from unlinked targets across the container.

The plurality of magnets may be supported by a housing. The plurality of magnets may be enclosed by the housing. The housing may have an interior frame defining a two-dimensional frame array and the magnetic elements are positioned in the two-dimensional frame array to define the two-dimensional magnetic array.

The magnetic separation apparatus may further comprise one or more guiding elements attached to the housing.

The container may comprisess at least one of a tube, a vial, a Petri dish, a bottle, a bag, and a multi-well micro-plate.

The multi-well micro-plate may comprise at least one of an isolated tube or single well, a 6-well plate, a 12-well plate, a 24-well plate, a 96-well micro-plate, a 384-well micro-plate, and a 1536-well micro-plate.

The plurality of magnets may comprise at least one of a plurality of permanent magnets and a plurality of electromagnets.

Also disclosed, but not claimed, is a method for magnetic separation comprising: providing a container comprising a plurality of targets; adding a plurality of magnetically susceptible particles to the container such that at least some of the targets become linked to the magnetically susceptible particles; and positioning the container adjacent to a magnetic field of a two-dimensional magnet array having a plurality of magnets arranged adjacent to each other in rows and columns of the array; wherein respective magnetic fields of magnets of said plurality of magnets in a first row of the array are arranged so as to define a first order Halbach array, and proceeding in a column direction of the array, respective magnetic fields of magnets of said plurality of magnet in each of a second and succeeding rows of the array are oriented in the same direction relative to respective magnetic fields of magnets of said plurality of magnets in any immediately preceding row, such that a plurality of magnetic poles of the plurality of magnets apply magnetic fields oriented perpendicular to the plane to substantially consistently separate the plurality of magnetically susceptible particles from the plurality of particles across the container.

Providing the container may comprise providing one of a tube, a vial, a Petri dish, a bottle, a bag, and a multi-well micro-plate.

Providing the multi-well micro-plate may comprise providing at least one of an isolated tube or single well, 6-well plate, 12-well plate, 24-well plate, 96-well micro-plate, a 384-well micro-plate, and a 1536-well micro-plate.

Arranging the plurality of magnets may comprise supporting the arranged plurality of magnets within a protective housing.

Arranging the plurality of magnets may comprise arranging at least one of a plurality of permanent magnets and a plurality of electromagnets.

A plurality of magnetic separation apparatuses defined in accordance with the teachings herein may be arranged to provide a substantially planar array with the two-dimensional arrays positioned adjacent one another to provide a larger planar Halbach array.

A pair of magnetic separation apparatuses defined in accordance with the teachings herein may be arranged back to back to enable separations in containers on either side of the pair of magnetic separation apparatuses.

At least two magnetic separation apparatuses defined in accordance with the teachings herein may be arranged in an at least partially facing relationship with a receiving area for a container between the at least two magnetic separation apparatuses.

The at least two magnetic separation apparatuses may be oriented such that a plane defining a magnetic field strength minimum is obtained near the plane defining the magnetic field strength midpoint between the at least two magnetic separation apparatuses.

It will be appreciated by a person skilled in the art that an apparatus, system or method disclosed herein may embody any one or more of the features contained herein and that the features may be used in any particular combination or sub-combination.

These and other aspects and features of various embodiments will be described in greater detail below.

DRAWINGS

For a better understanding of the described embodiments and to show more clearly how they may be carried into effect, reference will now be made, by way of example, to the accompanying drawings in which:

Figure 1 is a schematic of an example embodiment of a magnetic separation process;

Figure 2 is a simplified schematic of an example embodiment of a magnetic separation process using a plate magnet;

Figure 3A is an illustration of an example of magnetic field lines generated in an interpolar gap;

Figures 3B is an illustration of magnetic field lines generated using a quadrupole magnet configuration;

Figure 3C is an illustration of a Halbach magnet array in accordance with an example embodiment;

Figure 3D is an illustration of magnetic field lines for the Halbach magnet array of Figure 3C;

Figure 4 illustrates the magnetic field strength for various example configurations of magnet arrays;

Figure 5A is a top plan view of magnetic elements of a two-dimensional magnetic array in accordance with an example embodiment;

Figure 5B is a perspective view of a housing for a two-dimensional magnetic array in accordance with an example embodiment;

Figure 6A is a top plan view of magnetic elements of examples of magnet arrays with constant element size;

Figure 6B is a perspective view of the magnet arrays of Figure 6A;

Figure 7 is a chart showing magnetic field decay with height for various examples of magnet array configurations;

Figure 8A is a top view of the magnet arrays of Figure 6A with cell separation testing sites identified;

Figure 8B is a chart showing cell separation performance for the magnet arrays of Figure 6A at the cell separation testing sites shown in Figure 8A;

Figure 9A is a chart showing recovery versus time for various example magnet array configurations;

Figure 9B is a chart showing change in purity odds ratio versus time for various example magnet array configurations;

Figure 10A is a chart showing recovery versus time for various example magnet array configurations;

Figure 10B is a chart showing purity change in odds ratio versus time for various example magnet array configurations;

Figure 11 is a chart showing average MFD versus process volume height for various example magnet array configurations; and

Figure 12 is a chart showing an example magnetization curve of superparamagnetic particles;

Figure 13 is a simplified perspective view of two-dimensional magnetic arrays in an example embodiment of a magnetic separation apparatus;

Figure 14 is a simplified perspective view of two-dimensional magnetic arrays in another example embodiment of a magnetic separation apparatus;

Figure 15 is a top plan view of magnetic elements of examples of magnet arrays with varying element size;

Figure 16 is a simplified perspective view of two-dimensional magnetic arrays in a further example embodiment of a magnetic separation apparatus;

Figures 17A-17B are top plan views of two-dimensional magnetic arrays in various further example embodiments of a magnetic separation apparatus.

The drawings included herewith are for illustrating various examples of articles, methods, and apparatuses of the teaching of the present specification and are not intended to limit the scope of what is taught in any way.

## DETAILED DESCRIPTION

Various apparatuses or processes will be described below to provide an example of an embodiment of the claimed subject-matter. No embodiment described below shall be considered in a limiting sense and may cover processes or apparatuses that differ from those described below. The claimed subject-matter is not limited to apparatuses or processes having all of the features of any one apparatus or process described below or to features common to multiple or all of the apparatuses described below. It is possible that an apparatus or process described below is not an embodiment of any claimed subject-matter. Any subject-matter disclosed in an apparatus or process described below that is not claimed in this document may be the subject matter of another protective instrument, for example, a continuing patent application, and the applicants, inventors or owners do not intend to abandon, disclaim or dedicate to the public any such subject-matter by its disclosure in this document.

Furthermore, it will be appreciated that for simplicity and clarity of illustration, where considered appropriate, reference numerals may be repeated among the figures to indicate corresponding or analogous elements. In addition, numerous specific details are set forth in order to provide a thorough understanding of the example embodiments described herein. However, it will be understood by those of ordinary skill in the art that the example embodiments described herein may be practiced without these specific details. In other instances, well-known methods, procedures, and components have not been described in detail so as not to obscure the example embodiments described herein. Also, the description is not to be considered as limiting the scope of the example embodiments described herein.

The terms "an embodiment," "embodiment," "embodiments," "the embodiment," "the embodiments," "one or more embodiments," "some embodiments," and "one embodiment" mean "one or more (but not all) embodiments of the present invention(s)," unless expressly specified otherwise.

The terms "including," "comprising," and variations thereof mean "including but not limited to," unless expressly specified otherwise. A listing of items does not imply that any or all of the items are mutually exclusive, unless expressly specified otherwise. The terms "a," "an," and "the" mean "one or more," unless expressly specified otherwise.

As used herein, the wording "and/or" is intended to represent an inclusive - or. That is, "X and/or Y" is intended to mean X or Y or both, for example. As a further example, "X, Y, and/or Z" is intended to mean X or Y or Z or any combination thereof.

Magnetic cell separation uses a magnetic field to separate magnetically linked targets (also referred to as magnetically labelled cells) from a suspension. For simplicity and ease of exposition, the description may refer to magnetic separation techniques applied to suspensions, such as suspensions of cells (i.e. magnetic cell separation). However, it should be apparent to a skilled reader that the apparatuses, systems and methods described herein may also be used with other targets of interest, such as DNA, RNA, proteins etc. that can be linked with magnetically susceptible particles for magnetic separation. In general, embodiments of the magnetic separation apparatuses, systems and methods described herein may be used with any magnetically susceptible particles.

Magnetic cell separation technologies are continually being developed with an aim of providing sensitive, selective, rapid and high-yield cell separation. Two techniques that have been developed for magnetic cell separation are positive selection and negative selection. In positive selection methods, target cells are labelled with magnetic particles. In negative selection methods, undesired cells are labelled with magnetic particles.

The choice of a positive selection method versus a negative selection method may depend on the target type of interest and/or downstream applications involving the target type of interest. For example, positive selection methods may be preferred for isolating a specific target type from a population when the targets will be labelled directly with magnetic particles. However, positive selection may interfere with some downstream applications. For instance, positive selection can be problematic if the magnetic particles and/or antibodies block a binding or active site involved in a downstream study. In such cases, negative selection may be preferred as the undesired target types are labelled and removed from the population, leaving targets of interest untouched/unmodified. Magnetic separation can also be applied with various targets of interest, such as cells, DNA, RNA, and/or proteins that can be linked with magnetically susceptible particles.

In general, to perform magnetic separation, a suspension that includes targets (e.g. cells, DNA, RNA, and/or proteins) can be mixed with magnetic particles in a container. The magnetic particles can be selected/designed to link with a particular target type or target subpopulation (e.g. cells of interest in the case of positive selection methods or undesired cells in the case of negative selection methods). The mixed targets and magnetic particles can then be incubated to allow the magnetic particles to link with the particular target type(s) (referred to herein as linked particles, magnetic linked particles, magnetically labelled targets, and/or magnetically tagged targets). For example, the magnetic particles can be coupled with a specific antibody that recognizes a corresponding unique epitope on a particular cell type/subpopulation. The antibodies link the target cells and magnetic particles creating a cell-antibody-magnetic particle complex.

The targets of interest can then be recovered from the suspension by exposing the container to a magnetic field. The magnetic field induces the magnetically linked targets to accumulate at a container surface closest to the magnet surface. The unlabeled targets can then be extracted into a new container (e.g. by aspiration, pour-off, hydrophilic/capillary extraction, syringe pump, a gravity flow-cell system, a pump and/or a pressure gradient), leaving the magnetically linked targets in the original container, e.g. at the container surface near the magnet. Alternatively, the extracted targets may be the desired targets if a negative selection method is used.

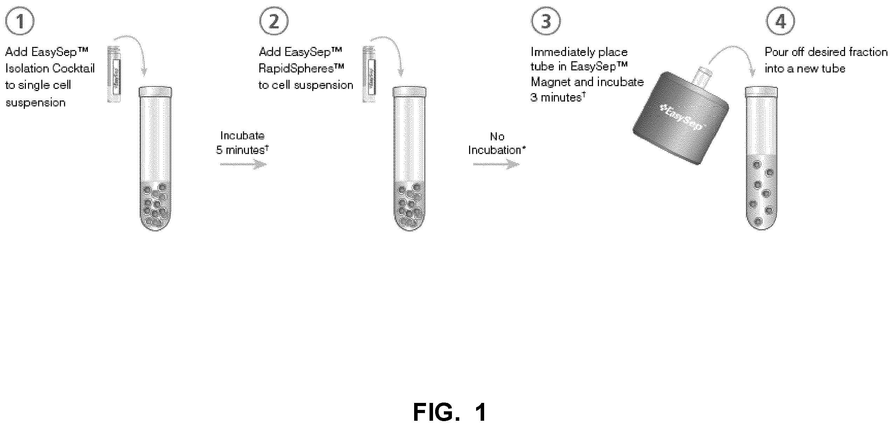

Figure 1 shows an example schematic of an embodiment of a magnetic separation process. One example of a magnetic cell separation process is referred to as an EasySep™ separation process from Stemcell Technologies. The magnetic separation shown in Figure 1 is an example embodiment using the EasySep™ separation protocol with a quadrupole magnet array. Reagent concentration, incubation time, and separation time vary depending on the desired separation and the strength of the magnet used.

In the example magnetic cell separation process, a cell suspension is provided in a container such as the tube shown in Figure 1. In other embodiments, different containers such as a vial, a Petri dish, a bottle, a deformable container (e.g. a bag), or a multi-well micro-plate may be used. In different examples using multi-well microplates, different micro-plate configurations may be used, such as a 6-well plate, a 12-well plate, a 24-well plate, a 96-well micro-plate, a 384-well micro-plate, and a 1536-well micro-plate.

The cell suspension may include a plurality of cells of interest and a plurality of undesired cells. A plurality of magnetic linking particles are introduced (i.e. added) to the cell suspension. The magnetic linking particles can be selected to link with one or more subpopulations of cells in the cell suspension such that the cells in that subpopulation, once linked with the magnetic linking particles, are magnetically susceptible. These linked particles may be referred to as a plurality of magnetically linked cell particles or magnetically linked particles. In some cases, the cell suspension and the magnetic linking particles may be incubated to allow the magnetically linked particles to form.

In some cases, the plurality of magnetic linking particles may be selected to link with the cells of interest (i.e. in the case of a positive selection method). In other cases, the plurality of magnetic linking particles may be selected to link with the undesired cells (i.e. in the case of a negative selection method).

Once the linked particles have been formed (e.g. after incubation), the container may then be positioned in close proximity to a magnet array as disclosed herein. For example, the container may be positioned adjacent to a surface of the magnet array. In some cases, the container may be guided into a position near the surface of the magnet array by guide elements provided by the magnet array and/or a housing for the magnet array. As shown in Figure 1, the container may be positioned within a receiving area provided by the housing for a quadrupole magnet array (this is not within the scope of the claims). The guide elements may include a wall or walls that guide the container into the receiving area, e.g. by being shaped to accommodate the container in the receiving area.

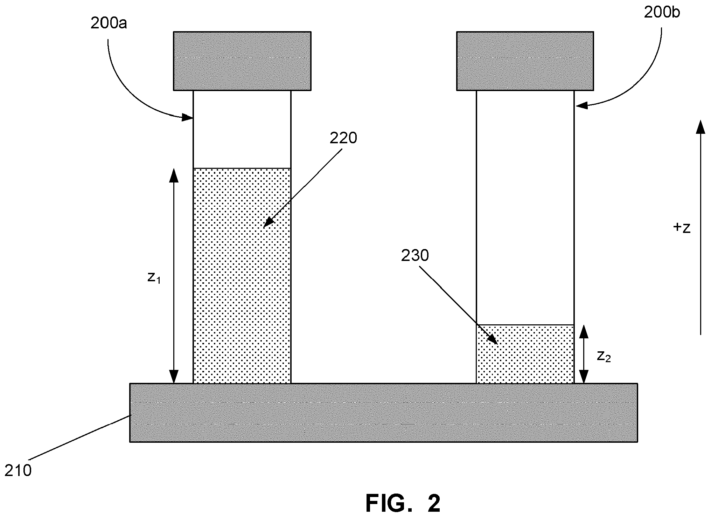

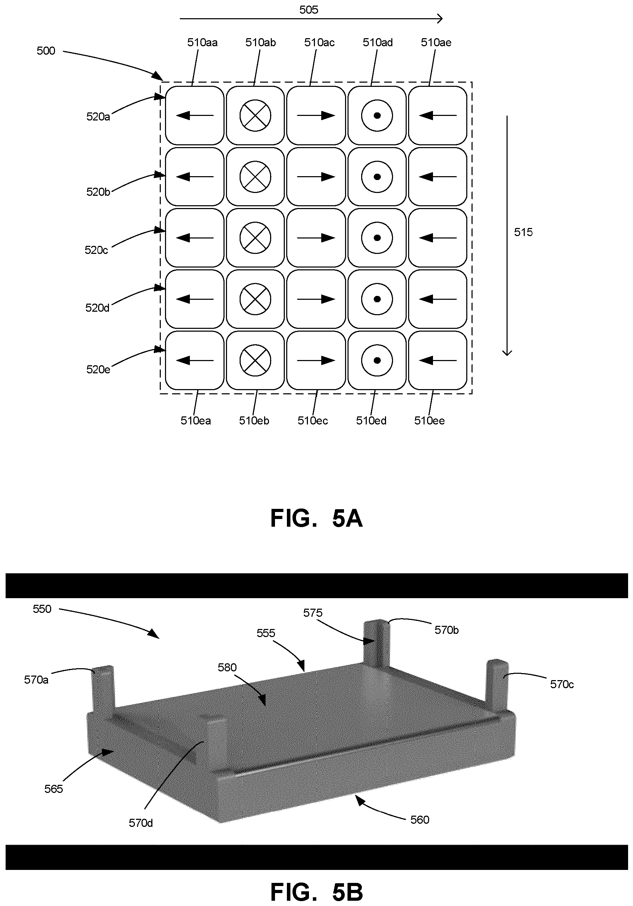

In the embodiments described herein using a two-dimensional magnet array, the container may be positioned in proximity to the array surface of the two-dimensional magnet array. For example, as shown in Figures 2 and 5B, the two-dimensional array may be provided as a plate magnet. The container may then be placed on a surface proximate the array surface (e.g. the array surface itself or a container receiving surface adjacent/just above the array surface).

An example of a container receiving surface is shown in Figure 5B.

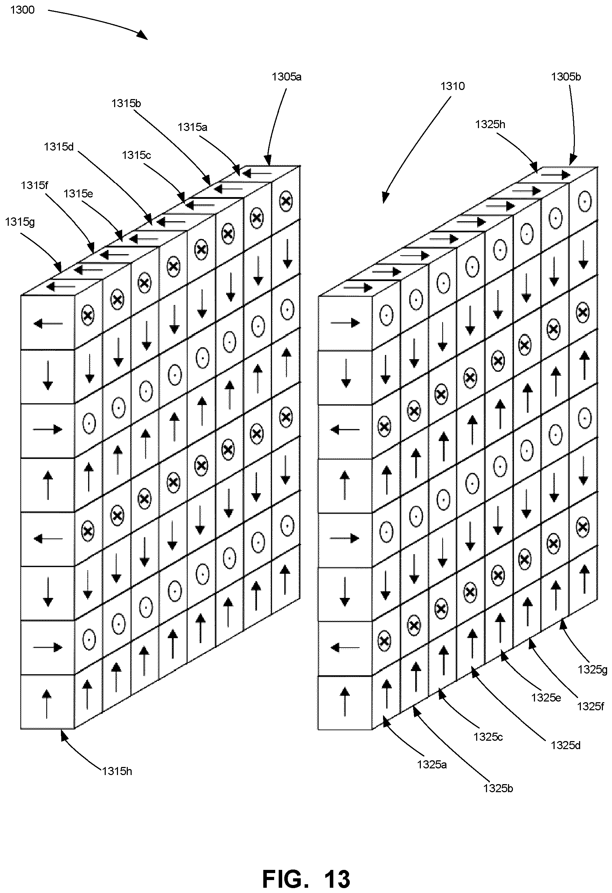

In some cases, the container may be positioned in a receiving area proximate to the array surface of one or more two-dimensional magnet arrays. Some examples of receiving areas are shown in Figures 13, 14, 16, 17A and 17B.

The container comprising the magnetically linked targets and unlinked targets may be left to incubate in proximity to the surface of the magnet array (e.g. in the receiving area). The magnetic field generated by the magnet array may attract the magnetically linked targets and magnetic particles to the container surface proximate the magnet array. The remaining, unlinked, targets may then be removed from the container e.g. by aspiration, pour-off, etc. As the unlinked targets are aspirated or poured off, the magnetically linked targets can be retained in the original container because of the magnetic field exerted by the magnet array.

In other embodiments, the unlinked targets may be removed from the suspension, e.g. through an outlet positioned adjacent the surface of the magnet array. This may be useful, for instance, in magnetic separations processes involving a flowing cell suspension. For instance, a suspension including magnetically labelled cells may be allowed to flow into a container positioned adjacent the surface of the magnet array. In such cases, the magnetically labelled cells may be removed from the flowing suspension as they pass the magnetic array and the unlinked targets may be allowed to continue to flow out of the flow cell.

The performance of a cell separation can be measured based on purity of the sample and the recovery of target cells after separation. Example purity and recovery parameters are defined in equations (1) and (2):

$$\text{Purity} = P_{\text{tar}} = \frac{N_{\text{tar}}}{N_{\text{tar}} + N_{\text{ntar}}} \tag{1}$$

$$\text{Recovery} = \frac{N_{\text{tar,final}}}{N_{\text{tar,inital}}} = \frac{N_{\text{final}} P_{\text{tar,final}}}{N_{\text{inital}} P_{\text{tar,initial}}} \tag{2}$$

where N is the number of cells, the subscript "tar" represents the number of target cells, the subscript "ntar" represents the number of non-target cells, the subscript "initial" represents the initial target cell fraction pre-separation, and the subscript "final" represents the cell fraction post-separation.

To achieve a fast recovery rate in a plate magnet, the average magnetic field gradient on the cells (over the entire process volume) should be large. In a plate magnet system the separation performance may depend on the strength of the magnet, the shape of the container holding the cell suspension, and the processed sample volume.

Figure 2 shows an example of the maximum process volume height, $z_1$, and the capture volume height, $z_2$ for an example plate magnet system. In the example of Figure 2, a container 200 is positioned adjacent a surface of the plate magnet 210. The container 200a is shown with the initial processed sample volume 220, including a first plurality of magnetically linked targets, a second plurality of unlinked targets, and a third plurality of unlinked magnetic particles. The processed sample volume 220 corresponds to the volume in container 200 after magnetic particles have been added and the suspension is allowed to incubate, but prior to the unlinked targets being removed. The container 200b is shown containing a capture volume 230, which corresponds to the amount of volume retained in the tube after aspiration of unlinked targets (i.e. the volume of the suspension that remains in container 200 after the unlinked targets are removed). In Figure 2, the separation distance is the difference between the maximum height, $z_1$, of the process volume 220 and the height, $z_2$ of the capture volume 230.

In the embodiments described herein, magnetic arrays are formed using a plurality of magnetic elements as sources of magnetic fields. Electromagnets and permanent magnets are examples of sources of magnetic fields that may be used as the magnetic elements in various embodiments described herein.

Electromagnets generally require a continuous supply of electrical energy to induce a magnetic field. To generate large magnetic fields (e.g. around 1T) using electromagnets, a large current is often required. However, a large current may cause heating of the sample and/or pose safety concerns.

Permanent magnets can generate magnetic fields passively. Advancements in permanent magnet technology may provide strong magnetic fields of 1T over volumes that may be relevant for cell separation methods (see, for example, M. Zborowski, Magnetic Cell Separation, Amsterdam: Elsevier, 2008). As a result, permanent magnets may provide a simple, affordable alternative for magnetic separation applications as compared to electromagnets in some embodiments. Permanent magnets can be arranged in different configurations so that field components from the permanent magnets add algebraically at a point in space. This property is called superposition and can be used to produce strong local fields and high gradients.

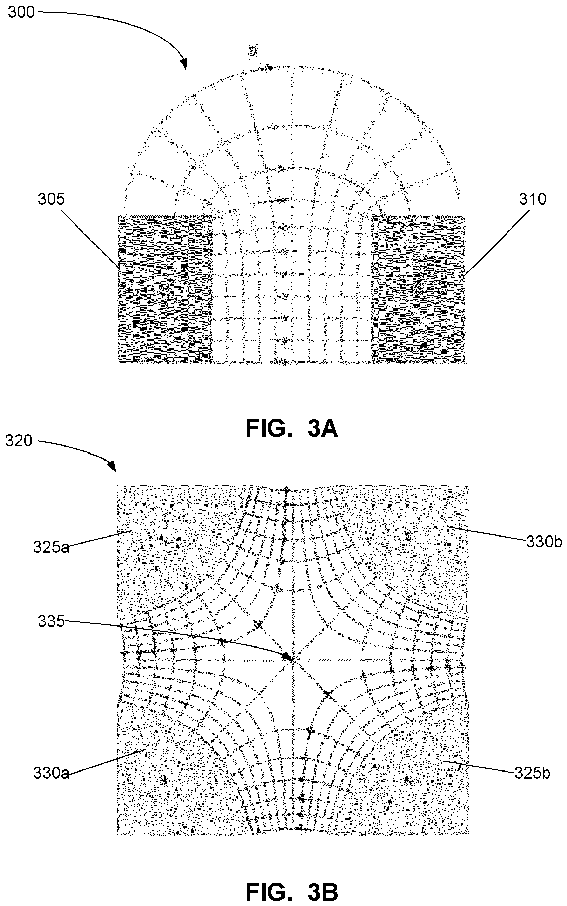

In prior systems, plate-type magnets typically use an array of alternating north and south magnetic elements (referred to herein as a Dexter Design). This configuration creates interpolar gaps that fringe the magnetic field, generating a large field gradient. An example of the magnetic field lines 300 of an interpolar gap between a North magnetic element 305 and a South magnetic element 310 are shown in Figure 3A.

In some other prior systems (e.g. those used with cylindrical geometries), a quadrupole circular arrangement 320 of magnetic elements 325 and 330 may be used as shown in Figure 3B. The quadrupole configuration 320 may provide a large magnetic field due to minimal loss of stray fields. The quadrupole configuration 320 may also provide a large field gradient because the field is large at the surface and cancels out to zero at the center 335 of the quadrupole magnetic arrangement 320.

While the quadrupole magnet configuration 320 may be useful for separations in a cylindrical geometry, the alternating north south arrangement 300 for planar magnet arrays may have some drawbacks. One potential disadvantage of the alternating N/S configuration 300 is that the interpolar gaps may need to be carefully arranged such that they fall precisely under the wells (i.e. under container units) that are to be processed. Wells/containers that are not aligned over the interpolar gap may not experience the full magnetic gradient. As a result, magnetic separations in nonaligned wells may be inferior to magnetic separations in wells/containers falling directly above an interpolar gap. This may result in a different magnetic array being required for each different container unit geometry (e.g. for different multiwell plate geometry). This may also introduce the potential for the alignment of containers on the magnetic array to significantly impact magnetic separation performance.

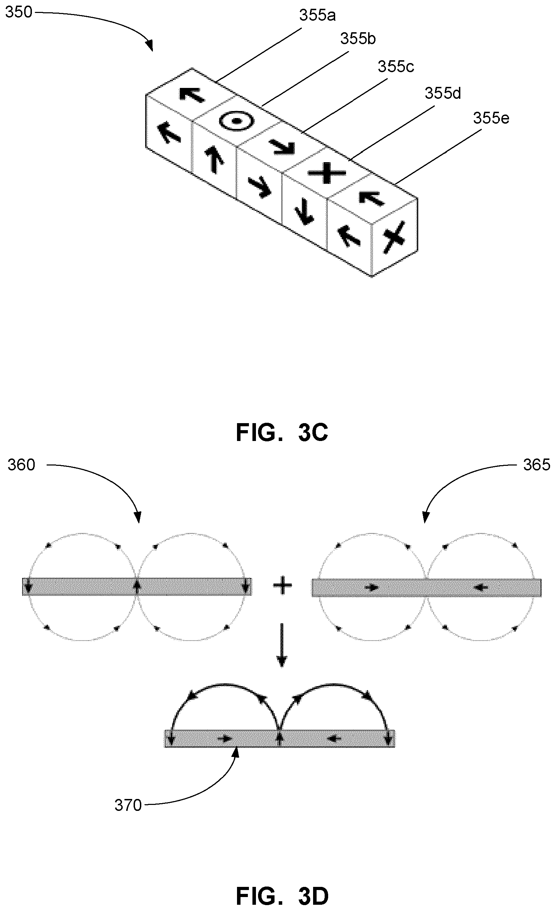

A Halbach array 350, seen in Figure 3C, has a rotating 90° pattern of magnetic elements 355a-355e. The Halbach array 350 uses the principle of superposition to amplify the magnetic field on one side while cancelling out the field on the other side, as illustrated in Figure 3D. The magnetic field 360 induced by magnetic elements 355a, 355c, and 355e is superposed with the magnetic field 365 induced by magnetic elements 355b and 355d to generate the magnetic field 370. As described in further detail herein below, embodiments using Halbach arrays 350 may enable a more uniform magnetic field to be provided for a container size of interest than other magnet configurations.

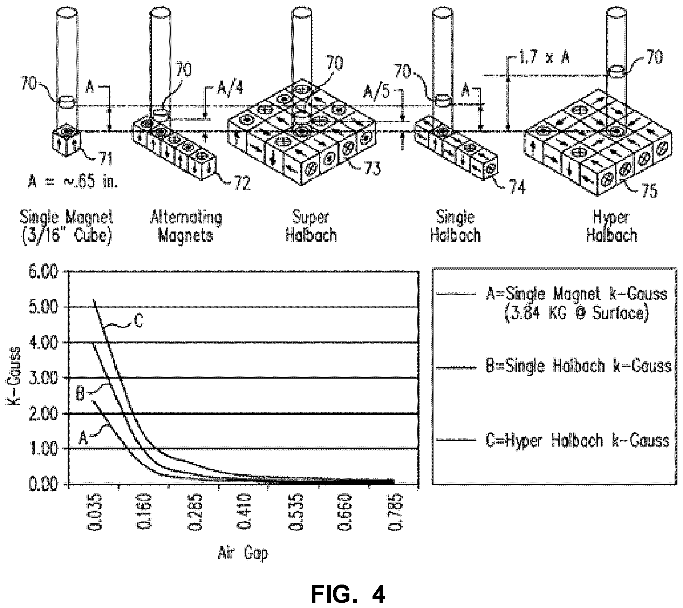

As shown in Figure 4, Halbach arrays have been used in a variety of different magnetic configurations, such as a Super Halbach (SH) configuration and a Hyper Halbach (HH) configuration. The plots illustrated in Figure 4 suggest that the HH configuration produces the relatively strongest magnetic field and steepest gradient, followed by a single Halbach configuration, and alternating magnet (i.e. North South) configuration.

Referring now to Figure 5A, shown therein is an example of a two-dimensional magnet array 500 that may be used in embodiments of the magnetic separation methods and apparatus described herein. The magnet array 500 is an example embodiment of a two-dimensional magnetic array that may be described as a linear Halbach array.

The magnet array 500 includes a plurality of magnetic elements 510 arranged to define a two-dimensional magnetic array.

The two-dimensional magnetic array includes a plurality of magnetic elements 510 extending along a first dimension 505 (also referred to as a row direction) and a plurality of magnetic elements 510 extending along a second dimension 515 (also referred to as a column direction).

In the example array 500, the plurality of magnetic elements 510 are arranged into a plurality of one-dimensional magnetic arrays 520. Each magnetic array 520 includes a plurality of magnetic elements 510 positioned adjacent one another extending in the direction of the first dimension 505 (i.e. to define a row of the array 500). The plurality of magnetic elements 510 in each one-dimensional magnetic array 520 are oriented to define a Halbach array such as the Halbach array shown in Figure 3C and described herein above.

For instance, the one-dimensional magnetic array 520a includes magnetic elements 510aa, 510ab, 510ac, 510ad, and 510ae. The orientation of magnetic elements 510aa, 510ab, 510ac, 510ad, and 510ae are rotated 90 degrees with respect to each adjacent magnetic element in the same one-dimensional magnetic array 520a.

Similarly, the one-dimensional magnetic array 520e includes magnetic elements 510ea, 510eb, 510ec, 510ed, and 510ee. The orientation of magnetic elements 510ea, 510eb, 510ec, 510ed, and 510ee are rotated 90 degrees with respect to each adjacent magnetic element in the same one-dimensional magnetic array 520e.

The magnetic elements 510 define an array surface of the magnetic array 500. In Figure 5A, the array surface is facing out from the page. The magnetic elements 510 are arranged such that the magnetic fields defined by the plurality of magnetic elements 510 attract magnetically susceptible particles towards the array surface.

In array 500, the magnetic fields defined by the plurality of one-dimensional magnetic arrays 520 are all oriented in the same direction along the surface of the magnetic array 500. In the example shown in Figure 5A, each one-dimensional magnetic array 520 has the same configuration and orientation of magnetic elements 510 as each of the other one-dimensional magnetic array 520. That is, each magnetic element 510 in array 500 has the same magnetic field orientation as each magnetic element that is adjacent to it in the direction of the second dimension 515 (i.e. each magnetic element 510 in a column has the same orientation).

In the example array 500 shown in Figure 5A, the magnetic elements 510 each have the same element size. In alternative embodiments, the size and shape of the magnetic elements in the magnetic array 500 may be varied and/or tuned based on the overall size of the array. For example, the size of magnetic elements 510 may vary between adjacent Halbach arrays 520 that still define magnetic fields oriented in the same direction at the surface of the array 500.

As shown in Figure 5A, the two-dimensional array 500 is substantially rectangular. In other embodiments, arrays may be configured into different two-dimensional configurations. In some cases, the shape of the magnetic elements may be adjusted to provide different shaped linear Halbach arrays.

In some cases, the surface of the array 500 may be substantially planar. In alternative embodiments, the surface of the array 500 need not be planar. For example, the surface of the array 500 may be shaped to conform to the shape of the container that is to be used with the magnetic separation apparatus. For instance, the surface of the array 500 may be curved in some examples. This may facilitate cell separation with containers that may also be curved such as bags or other deformable containers.

Embodiments described herein provide a magnetic separation apparatus. The magnetic separation apparatus may be used with a container configured to contain a plurality of targets. The plurality of targets can include desired cells/cells of interest and undesired cells, or DNA, RNA, or protein(s).

The plurality of targets may be linked with a plurality of magnetically susceptible particles to provide magnetically linked targets that are magnetically susceptible. In some cases, the magnetically linked targets may be magnetically susceptible particles. The magnetically linked targets may be generated, for instance, by linking targets (e.g. desired cells in the case of a positive selection technique or undesired cells in the case of a negative selection technique) and magnetic particles to create a magnetically linked target complex (or linked particles/cells) that includes the targets and magnetic particles.

The apparatus may include a plurality of magnetic elements arranged to define a two-dimensional magnetic array. For example, the magnetic array 500 shown in Figure 5A and discussed above may be used as the magnetic array in the magnetic separation apparatus.

In some examples, the magnetic elements can be arranged adjacent to each other defining a rectangular array having a plurality of rows and a plurality of columns (as in the example of array 500). In other embodiments, the magnetic array need not be rectangular.

In general, the magnetic elements in each row are arranged so as to define a first order Halbach array. The rows of magnets are arranged adjacent to one another such that the magnetic fields of each first order Halbach array are oriented in the same direction. The rectangular magnetic array can define a substantially planar magnetic plate. In some embodiments, each row of magnets in the substantially planar magnetic plate may provide substantially the same magnetic field.

In other words, the magnets in a first row of the rectangular array have respective magnetic fields arranged so as to define a first order Halbach array. Proceeding in a column direction of the array, respective magnetic fields of magnets in each of a second and succeeding row of the array are oriented in the same direction as respective magnetic fields of the magnets in any immediately preceding row.

Various different containers may be used with embodiments of the magnetic separation apparatus described herein. For instance, the container may be a multi-well micro-plate in some embodiments. The multi-well micro-plate may include at least one of an isolated tube or single well, a 6-well plate, a 12-well plate, a 24-well plate, a 96-well micro-plate, a 384-well micro-plate, and a 1536-well micro-plate. The container may also include at least one of a tube, a vial, a Petri dish, a bag (e.g. a soft-walled container of any shape) and a bottle for example.

In some cases, a container such as a tube or bag having an inlet or an outlet, or both, may allow a suspension comprising targets to flow while the magnetic separation method is performed. In other cases, the suspension may be substantially static within the container.

In various embodiments the magnets used in the magnetic separation apparatus may take the form of permanent magnets. This may avoid heating the sample being separated. This may also avoid safety concerns that may be associated with using currents to induce the magnetic fields.

In other embodiments, the magnets may be electromagnets. This may also the magnetic fields of the magnets to be controlled (e.g. activated and deactivated) connecting or disconnecting a current supply.

The magnets are supported by or within (i.e. enclosed by) a housing in the magnetic separation apparatus such as the housing 550 shown in FIG. 5B discussed below. The housing may facilitate construction and operation of the magnetic array.

Referring now to Figure 5B, shown therein is an example embodiment of a housing 550. A housing such as housing 550 is used to support a two-dimensional magnet array, such as magnet array 500 shown in Figure 5A. In some cases, the magnet array may be enclosed within the housing 550. The housing 550 may act as a protective housing around the magnet array 500. The housing 550 may also retain the magnetic elements of the magnet array 500 in place.

The housing 550 may include a housing base 560. The interior of the housing base 560 may define a frame for receiving the magnetic elements. The frame may include a plurality of channels and grooves that define the shape of a two-dimensional array. The housing base 560 may include walls defining the channels and grooves of the frame. The housing base 560 may also include sidewalls 565 extending upward from the housing base 560. In some cases, the sidewalls 565 may serve to define an outer portion of the channels in the housing base 560. Each channel may extend substantially across the array along a first dimension. Each channel may include a plurality of grooves corresponding to the positions along the first dimension at which magnetic elements may be positioned.

The magnetic elements of a magnet array can be positioned within the channels and grooves of the housing base 560. Individual magnetic elements may be secured in place within the corresponding channel and groove location. For example, a magnetic element may be secured to the housing base 560 using a set screw. The housing 550 may also include a housing cover 555 that may be mounted to cover the magnetic elements, and may or may not be magnetic, ferromagnetic, or magnetizable itself. In general, a thin non-ferromagnetic material is desired since it does not disturb the magnetic field. However, a ferromagnetic cover with a different surface profile may have positive effects on magnetic separation. The cover 555 may also provide additional support to maintain the magnetic elements in place. The housing cover 555 may also include a container receiving surface 580 adjacent the array surface of the magnet array supported by the housing. The container receiving surface 580 may be shaped to support a container for use in magnetic separation processes.

In embodiments where both are present, the housing base 560 may be thicker than the housing cover 555. This may allow the housing base 560 to provide structural integrity for the housing 550. The housing cover 555 may be thin to minimize interference with the magnetic fields of the magnet array and provide a maximum magnetic field strength for a container positioned adjacent to, or on, the container receiving surface 580 of the housing cover 555.

The housing 550 may facilitate manufacturing of the two-dimensional magnetic array, because the linear Halbach array structure can be an unstable magnetic configuration with adjacent magnetic elements repelling one another. In some embodiments, the magnetic elements may be formed of brittle materials, such as ceramic elements. In such cases, the housing 550 may be preferred to secure the magnetic elements in place while ensuring that the ceramic elements are not damaged.

Alternatively, less brittle materials, such as iron magnets, may be used in some embodiments. In such embodiments, the housing 550 may be omitted if an alternative method is used to fix the magnetic elements adjacent one another (e.g. glue or connectors between adjacent magnets).

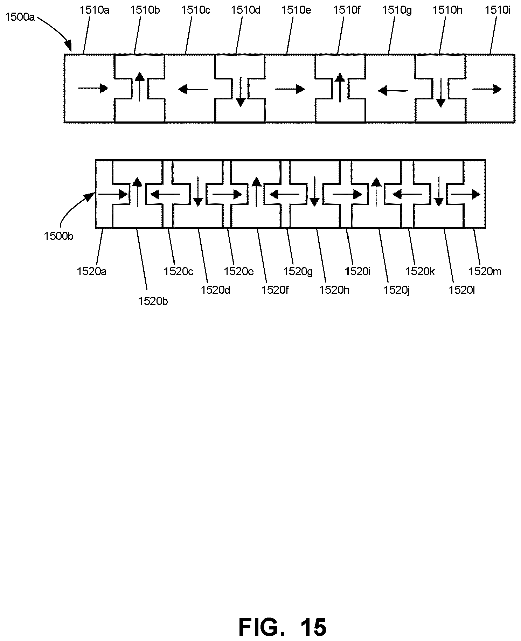

Additionally or alternatively, the magnetic elements may be shaped to interlock with one another (see e.g. Figure 15). In some cases, this may permit more brittle magnetic elements (e.g. ceramic elements) to be used without requiring the housing.

In some cases, the housing 550 may also include guide elements 570a-570d. The guide elements 570 may be used to guide or direct a container toward a container receiving surface 580 adjacent, or provided by, the top cover 555 of the housing 550. The guide elements 570 may also retain the container or container unit proximate the surface of the magnet array.

The guide elements 570 may include guide members that extend from the housing 550. The guide members may extend from the top cover 555 of housing 550, the sidewalls 565, and/or the housing base 560. The guide members may be shaped and positioned to correspond to the shape of a container used in a magnetic separation process. For example, the guide elements 570 may include guide members extending from the surface of the top cover of the housing at each corner of the housing 550. The guide elements 570 may include guide channels 575 shaped to engage a container and/or a container unit that holds a plurality of containers. In the example shown in Figure 5B, the guide elements may retain a multi-well container unit near the container receiving surface 580. This may facilitate multiple individual containers held within the multi-well container unit being concurrently positioned above, and proximate to, the container receiving surface 580.

In some cases, the guide elements 570 may also elevate the top cover 555 of the housing 550 from another surface if the magnetic separation apparatus is positioned with the top cover 555 facing a surface. This may facilitate removing the magnetic separation apparatus from magnetically susceptible surfaces, such as metal surfaces, by enabling the magnetic separation apparatus to be pried off the surfaces. The guide members may also operate as a safety feature to ensure that a user's hand is not crushed by the magnetic separation apparatus being attracted to a surface or to another magnetic separation apparatus.

The magnets in the array may be sized and arranged to provide a desired balance of field uniformity, field strength and magnetic field intensity decay rate (i.e. the useful depth in which a cell separation using magnetic particles may be achieved) for the container units and sizes of interest in various embodiments. The magnets may also be arranged to achieve a desired balance between field gradient and field penetration distance allowing for larger depth of magnetic separation volume for the container units and sizes of interest in various embodiments.

Embodiments of the magnetic separation apparatuses and systems described herein may also combine a plurality of two-dimensional magnetic arrays in a modular manner. The two-dimensional magnetic arrays may be assembled into larger arrays by combining individual two-dimensional magnetic arrays into larger two-dimensional magnetic arrays. This may allow the magnetic separation apparatus to be used with containers of different size. In embodiments described herein, the two-dimensional magnet arrays may include columns of magnetic elements having the same orientation, while the rows define Halbach arrays. This configuration may facilitate a modular construction in which multiple two-dimensional magnet arrays can be positioned side-by-side with the magnetic fields oriented and facing in the same direction. As the columns have a repeating pattern/sequence of magnetic elements (e.g. the same element throughout the column), the columns can be easily extended across multiple two-dimensional magnet arrays.

In some cases, individual two-dimensional magnetic arrays can be arranged back to back so that separations can be performed on 2 sets of samples, such as in a tube rack configuration. This may facilitate rapid, automated processing methods, such as those involving automated pipetting systems. For instance, containers such as test tubes may be positioned on either side of the back to back two-dimensional magnetic arrays. Samples can be transferred to the tubes on either side, and a magnetic separation process can be performed on those samples substantially concurrently. Because the linear Halbach array results in a minimal magnetic field on the side opposite the array surface (as shown in Figure 3D above), minimal interference may occur in this back-to-back arrangement.

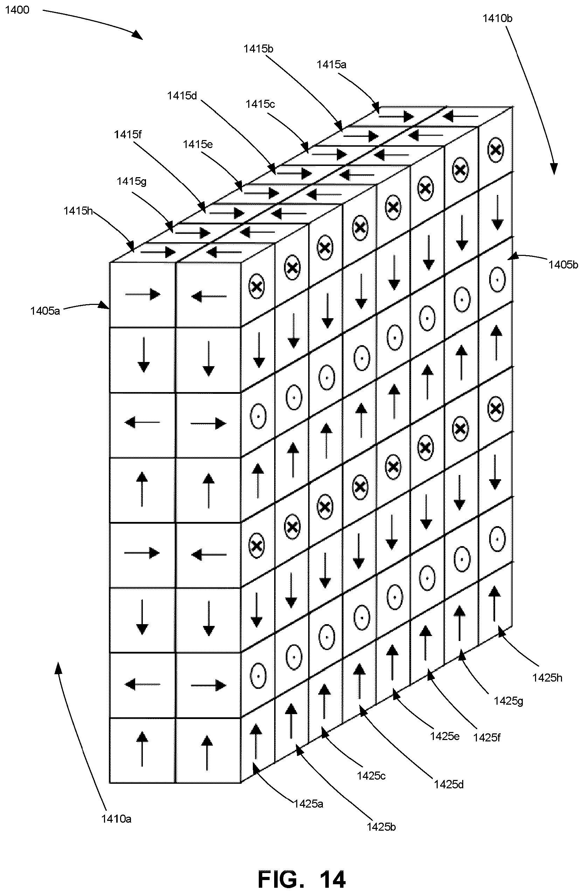

Referring now to Figure 14, shown therein is an example of a magnetic separation apparatus 1400. The magnetic separation apparatus 1400 includes a first two-dimensional magnet array 1405a and a second two-dimensional magnet array 1405b. The first two-dimensional magnet array 1405a includes a plurality of one-dimensional magnetic arrays 1415a-1415h. The second two-dimensional magnet array 1405b includes a plurality of one-dimensional magnetic arrays 1425a-1425h.

The apparatus 1400 includes a first receiving area 1410a corresponding to the first two-dimensional magnet array 1405a. The apparatus 1400 also includes a second receiving area 1410b corresponding to the second two-dimensional magnet array 1405b. This may allow containers to be positioned on either side of the apparatus 1400 for magnetic cell separation.

The pair of two-dimensional magnet arrays 1405a and 1405b are positioned in a back-to-back arrangement. That is, the magnetic field generated by the first two-dimensional magnet array 1405a is oriented in the direction opposite to the magnetic field generated by the second two-dimensional magnet array 1405b. Containers may be used on either side, or both, of the back-to-back arrays 1405a and 1405b to perform magnetic separation methods.

As the magnetic fields of the two-dimensional magnet arrays fall substantially to zero in the back surface of the magnet array, the magnetic separation apparatus 1400 may allow each of the first two-dimensional magnet array 1405a and the second two-dimensional magnet array 1405b to perform magnetic cell separation substantially simultaneously with containers positioned on opposite sides of the apparatus 1400.

In some cases, 2 or more of the two-dimensional magnetic arrays may be arranged in a facing arrangement (or partial facing arrangement). For example, 2 or more individual two-dimensional magnetic arrays can be arranged in a parallel manner with one or more containers positioned or sandwiched between planar arrays for magnetic separation.

Referring now to Figure 13, shown therein is an example embodiment of a magnetic separation apparatus or system 1300. The magnetic separation system 1300 includes a plurality of two-dimensional magnet arrays, including the first two-dimensional magnet array 1305a and the second two-dimensional magnet array 1305b. The two-dimensional magnet arrays 1305a and 1305b may be generally similar to two-dimensional magnet array 500 shown in Figure 5.

The first two-dimensional magnet array 1305a includes a plurality of one-dimensional magnetic arrays 1315a-1315h. The magnetic elements in each of the one-dimensional magnetic arrays 1315a-1315h are oriented to define a Halbach array. Furthermore, the magnetic fields of each Halbach array 1315a-1315h are oriented in the same direction in the first two-dimensional magnet array 1305a. The magnetic fields of magnetic elements laterally adjacent to one another, but in different Halbach arrays 1315 are also oriented in the same direction.

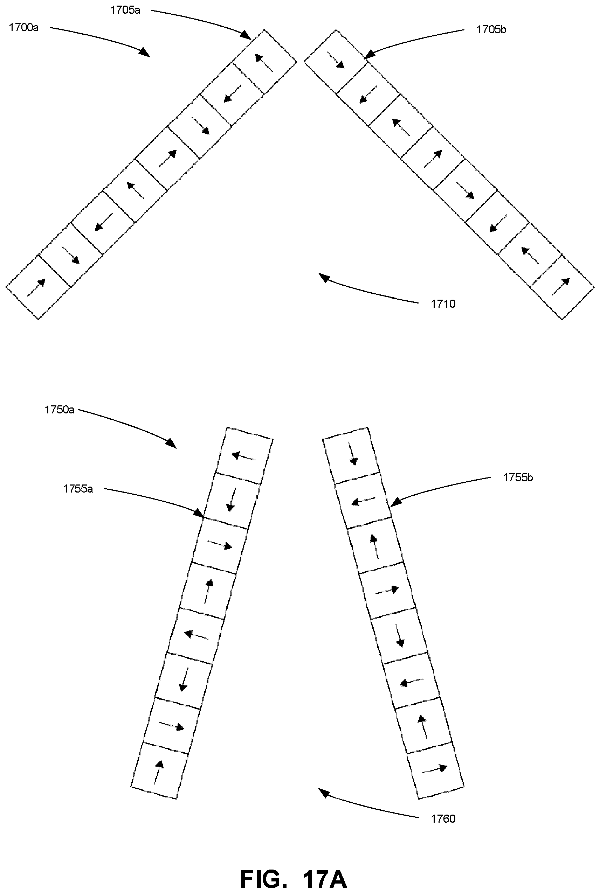

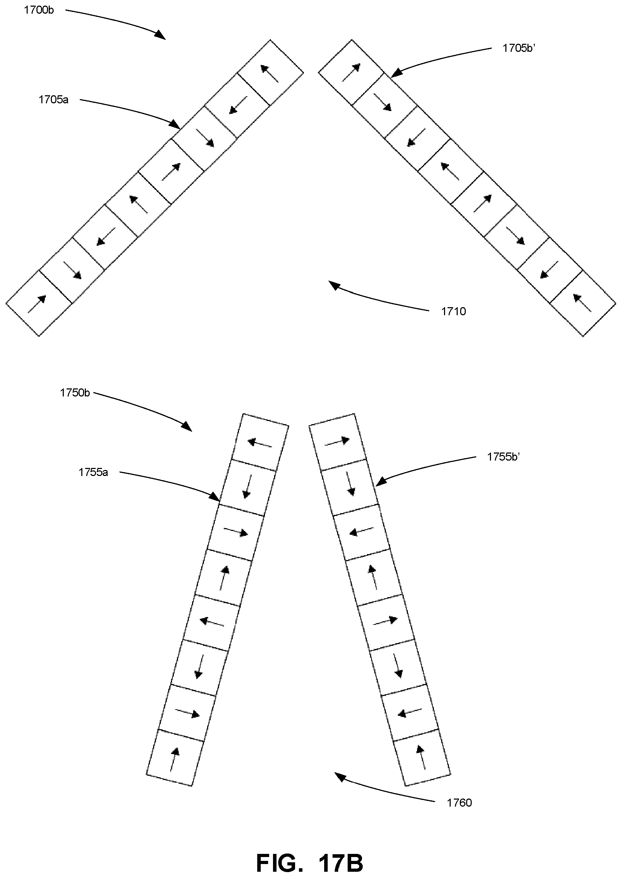

Similarly, the second two-dimensional magnet array 1305b includes a plurality of one-dimensional magnetic arrays 1325a-1325h. The magnetic elements in each of the one-dimensional magnetic arrays 1325a-1325h are oriented to define a Halbach array. Furthermore, the magnetic fields of each Halbach array 1325a-1325h are oriented in the same direction in the first two-dimensional magnet array 1305b. The magnetic fields of magnetic elements laterally adjacent to one another, but in different Halbach arrays 1325 are also oriented in the same direction. In other embodiments, the magnetic fields of laterally adjacent magnetic elements need not be in the same direction as shown in Figures 17A and 17B.

In system 1300, the pair of two-dimensional magnet array 1305a and 1305b are provided in a facing arrangement. That is, the magnetic field from the first two-dimensional magnet array 1305a is oriented towards the second two-dimensional magnet array 1305b. Similarly, the magnetic field from the second two-dimensional magnet array 1305b is oriented towards the first two-dimensional magnetic array 1305 a.

System 1300 includes a receiving area 1310. The receiving area is configured to receive a container. When a container is positioned in the receiving area 1310, a cell suspension within the container can be magnetically separated using the magnetic fields induced by the pair of two-dimensional magnet array 1305a and 1305b. For example, the receiving area 1310 may be configured to receive a test tubes, dishes or flasks or tray(s) of test tubes, dishes or flasks. Alternatively, the receiving area 1310 may positioned to allow a cell suspension to flow therethrough, allowing magnetically susceptible particles to be removed from the flowing suspension.

In the magnetic separation apparatus 1300, the first two-dimensional magnet array 1305a and the second two-dimensional magnet array 1305b are parallel to one another. The strength of the magnetic fields of the first two-dimensional magnet array 1305a and the second two-dimensional magnet array 1305b define a magnetic strength midpoint plane at a position within receiving area 1310 of the first two-dimensional magnet array 1305a and the second two-dimensional magnet array 1305b. The magnetic strength midpoint plane may correspond to the geometric midpoint, for instance, where the magnetic fields of the first two-dimensional magnet array 1305a and the second two-dimensional magnet array 1305b are identical. In other cases, where the magnetic field strength varies, the magnetic strength midpoint plane may not be positioned at the geometric midpoint. The magnetic fields of the first two-dimensional magnet array 1305a and the second two-dimensional magnet array 1305b can be selected to provide a magnetic field strength minimum substantially along the plane defining the magnetic strength midpoint. The receiving area 1310 may be configured to direct the container or containers to the magnetic strength midpoint plane. This may facilitate magnetic separation by providing a large field gradient between the first two-dimensional magnet array 1305a and the second two-dimensional magnet array 1305b.

The orientation between parallel planar arrays can be configured such that a plane defining a magnetic field strength minimum is obtained near the plane defining the magnetic field strength midpoint between the parallel planar Halbach arrays. The magnetic field strength midpoint may not correspond directly to the geometric midpoint between the two-dimensional magnetic arrays, for example where the magnetic field strength of the respective two-dimensional magnetic arrays is different. This may increase the magnetic field gradient as one moves away from the bisecting magnetic field minimum plane.

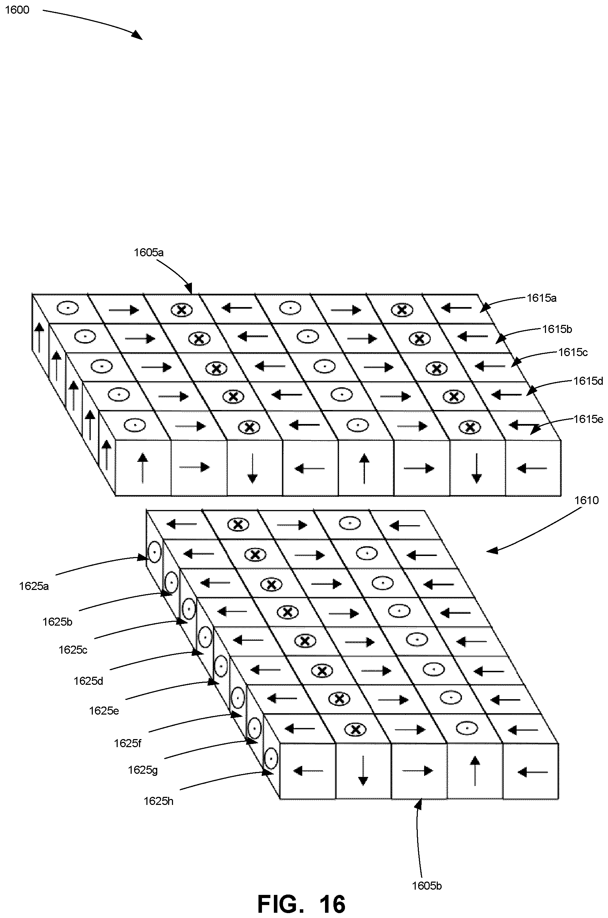

In some embodiments, the orientation of the magnetic fields of the facing two-dimensional magnetic arrays may be rotated with respect to one another (e.g. parallel, perpendicular, or other relative orientations). Referring now to Figure 16, shown therein is an example magnetic separation apparatus 1600. Magnetic separation apparatus 1600 includes a first two-dimensional magnet array 1605a and a second two-dimensional magnet array 1605b in a facing arrangement. A receiving area 1610 for a container is arranged between the first two-dimensional magnet array 1605a and the second two-dimensional magnet array 1605b.

The first two-dimensional magnet array 1605a includes a plurality of one-dimensional Halbach arrays 1615a-1615e. Similarly, the second two-dimensional magnet array 1605b includes a plurality of one-dimensional Halbach arrays 1625a-1625e. However, the orientation of the magnetic field of the first two-dimensional magnet array 1605a is rotated perpendicular with respect to the magnetic field of the second two-dimensional magnet array 1605b.

As well, the at least two facing two-dimensional magnetic arrays may be positioned at an angle to one another. The angle and rotation of the two-dimensional magnetic arrays can be adjusted depending on the magnetic field desired for a particular application.

Referring now to Figure 17A, shown therein are top plan views of magnetic separation apparatus 1700a and magnetic separation apparatus 1750a in accordance with example embodiments. Magnetic separation apparatus 1700a includes a pair of two-dimensional magnet arrays 1705a and 1705b in a perpendicular arrangement. A receiving area 1710 for a container is positioned in a region in front of both two-dimensional magnet arrays 1705a and 1705b. The magnetic fields of both two-dimensional magnet arrays 1705a and 1705b can thus overlap, at least partially, to attract targets in a container in the receiving area 1710.

Magnetic separation apparatus 1750a includes a pair of two-dimensional magnet arrays 1755a and 1755b in a partially facing arrangement (also referred to as an acute angular relationship). A receiving area 1760 for a container is positioned in a region in front of both two-dimensional magnet arrays 1755a and 1755b. The magnetic fields of both two-dimensional magnet arrays 1755a and 1755b can thus overlap, at least partially, to attract targets in a container in the receiving area 1760.

Referring now to Figure 17B, shown therein are top plan views of magnetic separation apparatus 1700b and magnetic separation apparatus 1750b in accordance with example embodiments. Magnetic separation apparatus 1700b is similar to apparatus 1700a in that it includes a pair of two-dimensional magnet arrays 1705a and 1705b' in a perpendicular arrangement. However, in magnetic separation apparatus 1700b the orientation of the two-dimensional magnet array 1705b' has been rotated with respect to the orientation of two-dimensional magnet array 1705b. As a result, the magnetic field interacting with a container in the receiving area 1710 would be different for magnetic separation apparatus 1700b as compared to magnetic separation apparatus 1700a.

Similarly, magnetic separation apparatus 1750b is similar to magnet separation apparatus 1750a except that the magnetic field of magnet array 1755b' is rotated with respect to the magnetic field of magnet array 1755b. Here again, the magnetic field interacting with a container in the receiving area 1760 would be different for magnetic separation apparatus 1750b as compared to magnetic separation apparatus 1750a.

The embodiments described herein may provide a substantially universal plate magnet that can be used for automation applications that can process small (e.g. 1 micro-Litre) to large volumes (> 50 mL) in a reasonable time frame.

To achieve maximum uniformity over a finite surface plane the size and shape of the magnetic blocks in the magnetic array (e.g. the LH array) can be varied and tuned dependent on the overall size of the array. For example, in an array of 85 mm x 130 mm (standard ANSI plate dimensions) alternating blocks of small cubic magnet elements can be assembled with larger (double size) blocks to reduce boundary effects of a finite linear Halbach array. In other examples, the magnetic elements may be rectangular (and not cubic) in shape or other shapes to provide the desired magnetic field distribution and uniformity.

Referring now to Figure 15, shown therein are examples of magnetic elements 1510 and 1520 that may be used to form one-dimensional Halbach arrays 1500a and 1550b respectively (and in turn two-dimensional magnetic arrays) in some embodiments described herein. A first one-dimensional Halbach array 1500a includes a first plurality of magnetic elements 1510a-1510i. A second one-dimensional Halbach array 1500b includes a second plurality of magnetic elements 1520a-1520i.

As shown in Figure 15, the magnetic fields of the magnetic elements 1510a-1510i are oriented to define the Halbach array 1500a. However, in the array 1500a, the magnetic elements 1510 do not have a constant element size. Rather, the size of the magnetic elements 1510 varies throughout the Halbach array 1500a. In the example array 1500a, the size of the magnetic elements 1510 varies in a repeating pattern, with each second magnetic element 1510 having the same size. Similarly, the magnetic elements 1510 do not have a constant shape. Rather, the shape also varies with each second magnetic element 1510 having the same shape. In Halbach array 1500a, magnetic elements 1510a, 1510c, 1510e, 1510g and 1510i have the same shape and size as one another. Magnetic elements 1510b, 1510d, 1510f and 1510h have the same shape and size as one another, but different from magnetic elements 1510a, 1510c, 1510e, 1510g and 1510i.

Halbach array 1500b also illustrates an example magnetic array using varying element size. As shown, the magnetic elements 1520b, 1520d, 1520f and 1520h have generally the same shape and size of magnetic elements 1510b, 1510d, 1510f and 1510h from array 1500a. However, the magnetic elements 1520a, 1520c, 1520e, 1520g and 1520i are smaller than the magnetic elements 1510a, 1510c, 1510e, 1510g and 1510i of array 1500a.

The Halbach arrays 1500a and 1500b also illustrate an example of one-dimensional magnetic arrays with interlocking magnetic elements. This may provide additional structural support between the magnetic elements in the arrays.

In some cases, magnetic field strength may be sacrificed in order to provide a more uniform magnetic field across the surface of the array for the container of interest. Thus, embodiments of the LH arrays described herein may provide both a strong magnetic field gradient and a very uniform field for a container unit of interest. This may enable, for example, cell separation to be carried out reproducibly for containers positioned at locations across the magnetic surface independent of the precise positioning of the sample on the surface of the magnet array.

The magnetic field is defined by magnetic field strength, $\boldsymbol{H}$, and magnetic field intensity, $\boldsymbol{B}$. The magnetic field strength vector describes the strength of the magnetic field source and the magnetic field intensity vector describes the resulting effect on the surrounding space. The magnetic field strength can be labelled as the driving force or "the cause", whereas the magnetic field intensity can be considered the coupled flux density or "the effect". In free space (a vacuum), the magnetic field intensity is proportional to the magnetic field strength as shown in equation (3).

$$\boldsymbol{B} = \mu_0 \boldsymbol{H} \tag{3}$$

where, $\mu_0$ is the magnetic permeability of free space $\left(\mu_0 = 4\pi \times 10^{-7}\ \frac{\text{T}\cdot\text{m}}{\text{A}}\right)$.

A magnetic field can be visualized using magnetic field lines. The magnetic field lines are tangent everywhere to the magnetic field strength and magnetic field intensity vectors. The presence of matter changes the relationship between magnetic field strength and magnetic field intensity. The magnetic field intensity in matter is given by equation (4).

$$\boldsymbol{B} = \mu_0(\boldsymbol{M} + \boldsymbol{H}) \tag{4}$$

$$\boldsymbol{m} = V\boldsymbol{M} \tag{5}$$

where, $\boldsymbol{M}$ is a magnetization vector. Magnetization defines the density of magnetic dipole moments $\boldsymbol{m}$ in a material of volume $V$, as expressed in equation (5).

In isotropic material the magnetization is proportional to the magnetic field strength. This property is defined in equation (6).

$$\boldsymbol{M} = \chi\boldsymbol{H} \tag{6}$$

where, $\chi$ is the volumetric magnetic susceptibility of the material.

Magnetic susceptibility is a dimensionless constant that describes the degree of magnetization of material in response to an applied magnetic field. Therefore, equation (4) can be rearranged as follows:

$$B = \mu_0(M + H) = \mu_0(\chi H + H) = \mu_0(\chi + 1)H = \mu_0\mu_m H \qquad (7)$$

where, $\mu_m$ is defined as the relative magnetic permeability of the material.

Magnetic susceptibility is often very small for biological material such as cells and their medium. This is because cells are composed of mostly water which is diamagnetic. Water has a very small, negative magnetic susceptibility on the order of $10^{-6}$. Ferromagnetic materials that may be used in magnetic cell separation can have magnetic susceptibilities on the order of $10^{-1}$. Thus, labelling target cells with magnetic particles can drastically increase the magnetic susceptibility of the target cell compared to its surroundings.

Magnetic force on a volume of magnetic material is given by equation (8).

$$F_\mathrm{m} = (m \cdot \nabla)B = V(M \cdot \nabla)B \qquad (8)$$

The magnetic force can be rewritten by substituting equations (6) and (7) into equation (8) yielding equation (9) below.

$$F_\mathrm{m} = V(M \cdot \nabla)B = V\chi(H \cdot \nabla)B = \frac{V\chi}{\mu_0(1 + \chi)}(B \cdot \nabla)B \qquad (9)$$

The total magnetic force on a magnetically labelled cell is given by equation (10).

$$F_\mathrm{m} = \frac{n_{\mathrm{p/c}}V_\mathrm{p}\chi}{\mu_0(1 + \chi)}(B \cdot \nabla)B \qquad (10)$$

where, $n_{p/c}$ is the number of particles per cell and $V_p$ is the volume of a single magnetic particle.

Therefore, the magnetic force on a labelled cell can be characterized by the magnetic particle properties (which are assumed constant), and the term $(B \cdot \nabla)B$, which is the differentiation of the magnetic field intensity vector, $B$, with respect to its self. This term is called the magnetic force density and is defined by the equation (11).

$$(B \cdot \nabla)B = \left\{ \begin{array}{l} \left(B_x \dfrac{\partial B_x}{\partial x} + B_y \dfrac{\partial B_x}{\partial y} + B_z \dfrac{\partial B_x}{\partial z}\right)\hat{\imath} \\[2ex] \left(B_x \dfrac{\partial B_y}{\partial x} + B_y \dfrac{\partial B_y}{\partial y} + B_z \dfrac{\partial B_y}{\partial z}\right)\hat{\jmath} \\[2ex] \left(B_x \dfrac{\partial B_z}{\partial x} + B_y \dfrac{\partial B_z}{\partial y} + B_z \dfrac{\partial B_z}{\partial z}\right)\hat{k} \end{array} \right\} \qquad (11)$$

In a one-dimensional case the magnetic force on a magnetically labelled cell can be simplified to equation (12).

$$F_{\mathrm{m,z}} = \frac{n_{\mathrm{p/c}}V_\mathrm{p}\chi}{\mu_0(1 + \chi)}B_z\frac{\mathrm{d}B_z}{\mathrm{d}z} \qquad (12)$$

The above formula shows that the magnetic force on a cell is a function of the magnetic field intensity and the magnetic field intensity gradient. The path of a labelled cell in an external magnetic field may not be intuitive, as it does not follow the magnetic field lines. The cell follows magnetic path lines defined by the magnetic force field defined above. The magnetic path lines are tangent everywhere to the magnetic force field.

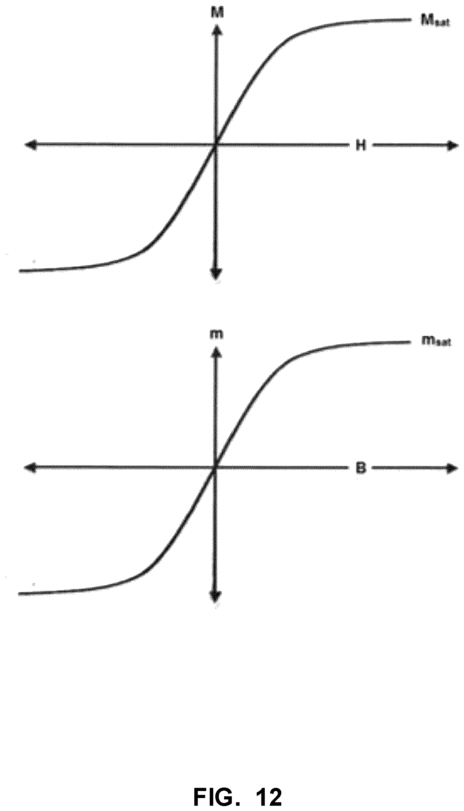

Magnetic nanoparticles typically used in cell separation are superparamagnetic. The magnetization curve of superparamagnetic particles can be seen in Figure 12. Figure 12 shows that the magnetic particle can either be in a linear superparamagnetic region where the magnetization is proportional to the magnetic field intensity or a saturated magnetic region where the magnetization remains constant. This can be mathematically represented by a term called effective susceptibility, $\chi_{\mathrm{eff}}$, in equation (13).

$$\chi_{\text{eff}} = \frac{\chi}{(1+\chi)} = \begin{cases} \dfrac{\mu_0 m_{\text{sat}}}{V B_{\text{sat}}}, & B < B_{\text{sat}} \\ \dfrac{\mu_0 m_{\text{sat}}}{V B}, & B \geq B_{\text{sat}} \end{cases} \tag{13}$$

Effective susceptibility can be substituted into equation (10), yielding equation (14) below.

$$\boldsymbol{F_{\text{m}}} = \frac{n_{\text{p/c}} V_{\text{p}} \chi}{\mu_0 (1+\chi)} (\boldsymbol{B} \cdot \nabla) \boldsymbol{B} = \frac{n_{\text{p/c}} V_{\text{p}} \chi_{\text{eff}}}{\mu_0} (\boldsymbol{B} \cdot \nabla) \boldsymbol{B} \tag{14}$$

The magnetic force on the target may depend on if the particles are in the linear or saturated superparamagnetic region. This can be represented in equation form by substituting the piecewise effective susceptibility equation (13) into equation (14), yielding equation (15). Simplifying equation (15) to a one-dimensional case gives equation (16).

$$\boldsymbol{F_{\text{m}}} = \frac{n_{\text{p/c}} V_{\text{p}} \chi_{\text{eff}}}{\mu_0} (\boldsymbol{B} \cdot \nabla) \boldsymbol{B} = \begin{cases} \dfrac{m_{\text{sat}}}{B_{\text{sat}}} (\boldsymbol{B} \cdot \nabla) \boldsymbol{B}, & B < B_{\text{sat}} \\ \dfrac{m_{\text{sat}}}{B} (\boldsymbol{B} \cdot \nabla) \boldsymbol{B}, & B \geq B_{\text{sat}} \end{cases} \tag{15}$$

$$F_{\text{m,z}} = \frac{n_{\text{p/c}} V_{\text{p}} \chi_{\text{eff}}}{\mu_0} B_z \frac{dB_z}{dx} = \begin{cases} \dfrac{m_{\text{sat}}}{B_{z,\text{sat}}} B_z \dfrac{dB_z}{dz}, & B_z < B_{z,\text{sat}} \\ \dfrac{m_{\text{sat}}}{B_z} B_z \dfrac{dB_z}{dz} = m_{\text{sat}} \dfrac{dB_z}{dz}, & B_z \geq B_{z,\text{sat}} \end{cases} \tag{16}$$

Equation (16) shows that, in the linear superparamagnetic region, the magnetic force is a product of the magnetic properties of the particles (which are constant), the magnetic field, and the magnetic field gradient. In the saturated superparamagnetic region the magnetic force is a product of the magnetic properties of the particles and the field gradient.

In order to have a fast recovery rate in a plate magnet, the average magnetic force on the targets in a container (over the entire process volume) should be large. This means that in the plate magnet system the separation performance will often depend on the hardware used, and the sample volume being processed. This is because the hardware and the process volume define the separation height range. The separation height range, seen in Figure 2, is the difference between the maximum process volume height, $z_1$, and the capture volume height, $z_2$. The capture volume being the amount of volume retained in the tube. In this section, the average magnetic force (over a given separation height range) will be derived for a uniform plate magnet system.

The magnetic nanoparticles available from STEMCELL Technologies used to evaluate the example plate magnet embodiments saturate at a magnetic field intensity of approximately 0.5T. In the plate magnet embodiments tested there are considerably more magnetic nanoparticles in the superparamagnetic linear region compared to the saturated region. This is because the maximum magnetic field intensity for the prototypes of the embodiments tested was around 0.5T at the surface which was found to decay exponentially with height. Therefore, the proportion of cells in the saturated region versus cells in the linear region is negligible. Thus, in embodiments of the plate magnet system the magnetic force on the cells can be given by equation (17). In a simplified one-dimensional case the magnetic force can be given by equation (18). The magnetic particle properties and labelling conditions in the linear superparamagnetic region are represented by a constant, y, defined in equation (19).

$$\boldsymbol{F_{\text{m}}} = \frac{n_{\text{p/c}} V_p \chi_{\text{eff}}}{\mu_0} (\boldsymbol{B} \cdot \nabla) \boldsymbol{B} = \frac{m_{\text{sat}}}{B_{\text{sat}}} (\boldsymbol{B} \cdot \nabla) \boldsymbol{B} = \gamma (\boldsymbol{B} \cdot \nabla) \boldsymbol{B} \tag{17}$$

$$F_{\text{m,z}} = \gamma B_z \frac{dB_z}{dz} \tag{18}$$

$$\gamma = \frac{n_{\mathrm{p/c}} V_p \chi_{\mathrm{eff}}}{\mu_0} = \frac{m_{\mathrm{sat}}}{B_{\mathrm{sat}}} \tag{19}$$

The magnetic field intensity as a function of height was determined experimentally for uniform plate magnets. The magnetic field decay with height for a uniform plate magnet is defined by equation (20). Where $\alpha$ is a positive constant that represents the magnetic field at the surface of the magnet, and $\beta$ is a positive constant that defines the magnetic field decay rate with height. Taking the derivative with respect to height of equation (20) gives equation (22), the magnetic field gradient as a function of height.

$$\boldsymbol{B} = B_x \hat{\boldsymbol{\imath}} + B_y \hat{\boldsymbol{\jmath}} + B_z \hat{\boldsymbol{k}} = \alpha e^{-\beta z} \hat{\boldsymbol{k}} \tag{20}$$

$$B_z = \alpha e^{-\beta z} \tag{21}$$

$$\frac{d\boldsymbol{B}}{dz} = \frac{dB_z}{dz} \hat{\boldsymbol{k}} = -\alpha \beta e^{-\beta z} \hat{\boldsymbol{k}} \tag{22}$$

$$\frac{dB_z}{dz} = -\alpha \beta e^{-\beta z} \tag{23}$$

The general magnetic force density function defined earlier in equation (11) can be simplified to equation (24) for the uniform plate magnet case.

$$(\boldsymbol{B} \cdot \nabla)\boldsymbol{B} = B_z \frac{dB_z}{dz} \hat{\boldsymbol{k}} = -\alpha^2 \beta e^{-2\beta z} \hat{\boldsymbol{k}} \tag{24}$$

Therefore, the magnetic force at a given height over a uniform plate magnet may be determined using equation (25).

$$\boldsymbol{F_{\mathrm{m}}} = -\alpha^2 \beta \gamma e^{-2\beta z} \hat{\boldsymbol{k}} \tag{25}$$

The negative sign indicates that the force would be acting in downward in the z direction (refer to Figure 2 for frame of reference). Equation (26) defines the magnetic force density magnitude (MFD) as the function fd(z). Taking the integral of equation (26) over the separation height range and dividing by the separation height range gives equation (27), the average MFD (AVG MFD).

$$\mathrm{fd}(z) = |(\boldsymbol{B} \cdot \nabla)\boldsymbol{B}| = \alpha^2 \beta e^{-2\beta z} \tag{26}$$

$$\mathrm{fd}_{\mathrm{avg}}(z_1) = \frac{\alpha^2}{2} \frac{\left(e^{-2\beta z_2} - e^{-2\beta z_1}\right)}{z_1 - z_2} \tag{27}$$

The AVG MFD is proportional to the average force experienced on the cells; therefore it is directly related to the recovery rate. As a result, the inventors used the AVG MFD as a parameter for comparing the various embodiments described herein.

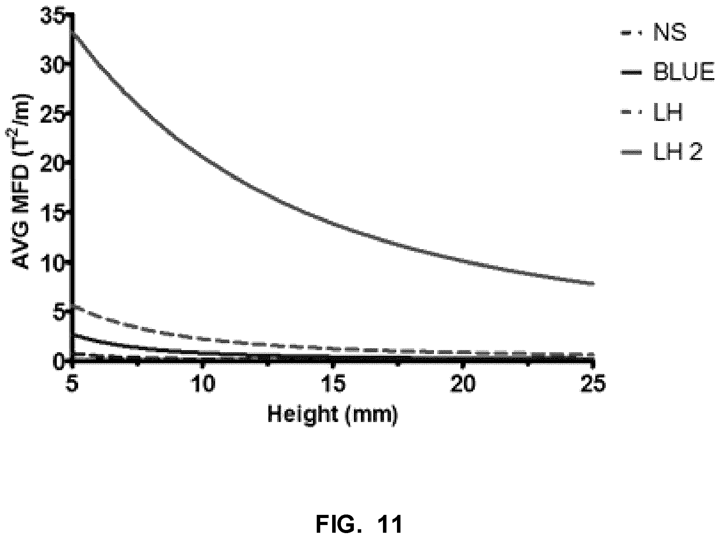

In view of the foregoing, the inventors identified that the performance of a given magnetic array is predictable by calculating the magnetic field density (MFD). For example, with ½" N52 magnets, an approximately 6-fold stronger array can be created as compared to an array constructed with ⅝" N52 magnets. As shown in Figure 11 a 6-fold stronger magnetic array (LH2) is achieved resulting in an increase of the separation zone (Z2) of approximately 25 mm - this increase makes separations in deep well plates possible (see, for example, LH2 shown in Figure 11).

The embodiments described herein may be implemented to satisfy various design objectives. First, it may be preferable that a magnet be compatible with a variety of hardware. A flat profile of a plate magnet may allow for easy compatibility with a large range of hardware such as microplates, tubes, cell culture flasks, or bags. Or, a curved or non-planar profile of the plate magnet may allow for easy compatibility with deformable containers, such as bags. Some example embodiments of the magnetic separation apparatuses described herein using two-dimensional magnet arrays may provide these benefits.

Embodiments described herein may also be mountable onto automated cell separation instruments. Embodiments described herein can, however, also have a large average MFD over a large volume range. The MFD depends on the magnetic field at the surface, the magnetic field gradient, and the separation height range. A uniform magnetic field is preferred so that separation performance is consistent at any location and with any hardware. Some embodiments described herein may be able to process samples in a time period of 15 minutes or less.

The recovery and final purity of targets separated using the described plate magnets may vary with hardware and process volume, however it is preferred that performance be at least equal to or greater to the performance of other magnet configurations against which the prototype embodiments were tested. The unit-size plate magnet should preferably have a footprint equal to the area of a standard microplate or about 0.011sq m (0.12 sq ft). Some example embodiments of the magnetic separation apparatuses described herein using two-dimensional magnet arrays will be handled by laboratory technicians. Accordingly, they may be implemented with a reasonable weight and necessary safety features such as the guide members described above. Furthermore, as described above embodiments of the magnetic separation apparatus disclosed herein may be easy to manufacture at a reasonable cost. For example, a housing with a frame may simplify construction of the magnetic separation apparatus.

Table 1: Design Objectives and Benchmark Values for Embodiments of the Plate Magnet

| Design Objective(s) | Benchmark Value(s) for Example Embodiments |
|---|---|
| Compatibility with a variety of hardware | Microplates, tubes, flasks, bags, etc. |
| Mountability | RoboSep-16 |
| Large average magnetic force over large height range | Greater than or equal to the BLUE magnet available from Stemcell Technologies (although it will be variable depending on associated hardware) |
| Uniformity of magnetic field | Consistent performance |
| Sample processing in reasonable time frame | Less than or equal to 15 minutes |
| High recovery of target cells | Greater than or equal to the BLUE magnet available from Stemcell Technologies (although it will be variable depending on associated hardware) |
| High final purity of target cells | Greater than or equal to the BLUE magnet available from Stemcell Technologies (although it will be variable depending on associated hardware) |
| Small footprint | .12 sq ft $\leq$ footprint $\leq$ 1 sq ft |
| Movability | $\leq$ 20lbs |
| Easy to manufacture | Simple and safe assembly |
| Reasonable cost to manufacture | $\leq$ \$400 |
| Safe to handle | Necessary safety features |

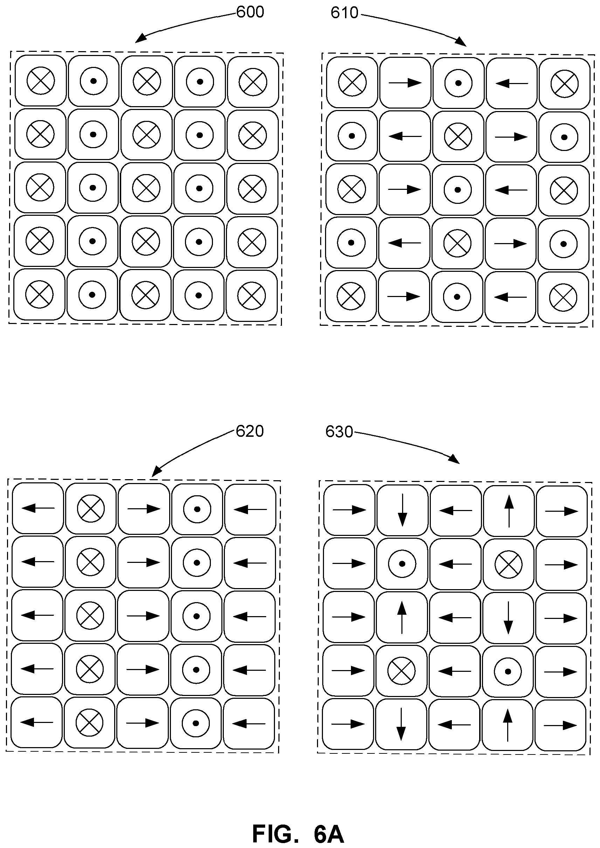

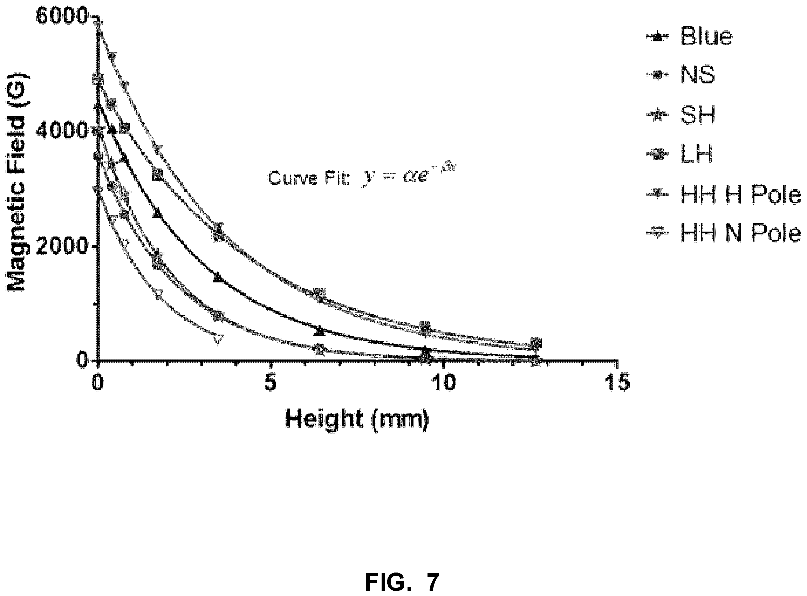

Three alternative configurations (shown in Figure 6A) of a plate magnet were tested and evaluated using prototypes constructed by the inventors. The alternative configurations tested were an example of the Linear Halbach (LH) array configuration 620 similar to the array 500 described herein shown in Figure 5A above, and the Super Halbach (SH) 610 and Hyper Halbach (HH) 630 arrays mentioned above. Prototypes of these embodiments were evaluated against existing NS plate magnet configurations 600 (Dexter Design) available from Stemcell Technologies. Desirable parameters such as consistent cell separation performance, large average MFD (Magnetic Force Density), high final recovery, high final purity, and ease of assembly were evaluated as a means of comparison, the results of which are shown in Figures 7 and 8B-11.

The magnetic testing experiments illustrated in Figure 4, suggest that the HH embodiment may produce the strongest magnetic field and steepest gradient, followed by the single Halbach, alternating magnets, and the SH. While the HH may provide a strong magnetic field it has a complicated assembly.

The SH design 610 tested by the inventors had consistent separation performance, but had an AVG MFD (for typical cell separation height ranges) similar to the Dexter NS design 600. Accordingly, the cell separation performance in the SH 610 was similar to the NS 600.

The LH embodiment 620 and HH configuration 630 tested had similar AVG MFD's (for typical cell separation height ranges) that were much greater than the NS configuration 600. This translated to the LH embodiment 620 and HH configuration 630 having similar cell separation performance in terms of final cell recovery and purity.

The LH embodiment 620 and HH configuration 630 had recovery rates that were approximately twice that of the NS configuration 600. This resulted in higher final recoveries and purities for the LH array 620 and HH array 630 compared to the NS configuration 600.

The LH embodiment 620 tested had a uniform magnetic field for the locations of interest, which resulted in consistent cell separation performance at all locations on the magnet. The HH configuration 630 tested did not show a uniform magnetic field and had a strong pole (HH H Pole) and a weak pole (HH N Pole). As a result, in the HH configuration 630 tested the cell separation performance was not consistent over all locations.

The LH embodiment 620 also provided a simple arrangement of magnets that facilitated assembly (as described above). The LH embodiment 620 was even found to outperform the current DEXTER NS design 600 magnet despite having significantly smaller and lower grade magnets.

A constant element size nine-by-nine array for each of the HH, LH and SH arrays were constructed using 1/4 inch cube N42 rare earth magnets. A top view schematic of the HH 630, LH 620 and SH 610 arrays is shown in Figures 6A along with the top view of a NS control configuration 600. The arrows point from the south pole to north pole of the magnet.

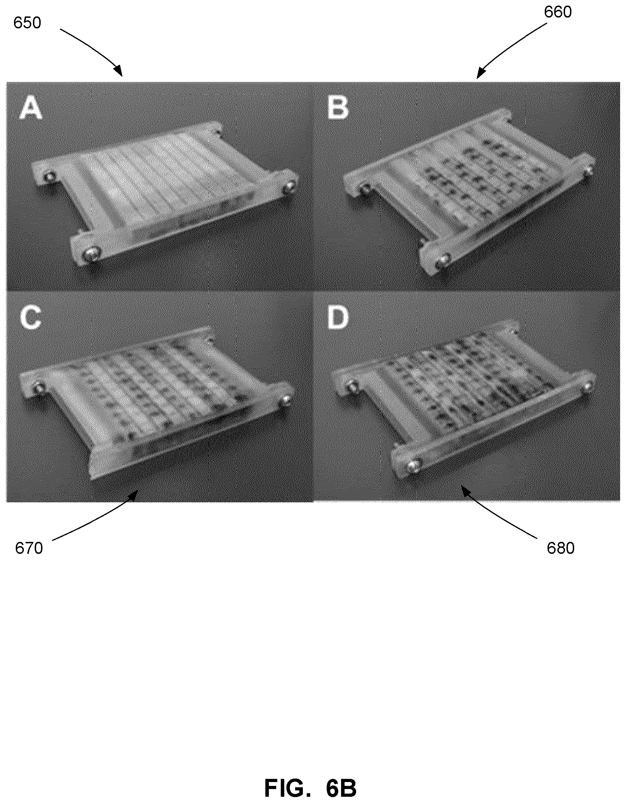

An example of a housing was used to hold the cube magnets in the desired orientation. Magnets were equally spaced apart in the X direction with 1mm ribs and in the Y direction with 1 mm plastic spacers. This may help maintain a uniform magnetic field. The top cover of the housing can be preferably as thin as possible (1 mm) to maximize the magnetic field at the surface. The housing base can have an increased thickness (3 mm) that may provide the housing with sufficient rigidity. A pair of housing portions (i.e. a base and top cover) may be used to lock the magnets in place and are shown in Figures 5B and 6B.

Multiple experiments were conducted to characterize the magnetic properties and separation performance of the prototypes of the alternative magnet array configurations. A gauss meter (AlphaLabs Inc. GM2-Model) was used to characterize the magnetic field for each magnet. CD3 positive cell selection separations were used to test prototype performance. Initial and final cell concentration measurements of live cells and CD3 positive cells were made by flow cytometry (BD Accuri™ C6). From these experiments, separation parameters such as the final recovery and logit purity difference could be calculated. The data gathered from these experiments was used to evaluate the performance of the prototypes for the example alternative plate magnet embodiments.

The magnetic field decay with height was characterized for each prototype by measuring the field at different height intervals (as shown in Figure 7). The NS, SH, LH, HH, and Dexter design (a BLUE magnet from Stemcell technologies) magnets were evaluated. In previous tests, it was discovered that the HH configuration may not be uniform. The HH configuration may have strong poles, which are referred to as Hyper Poles (H Pole), and weak poles called the Null Poles (N Pole). The HH H Pole and HH N Pole were each tested in this experiment. Measurements were made at 0, 0.4, 0.76, 1.72, 3.46, 6.4, 9.46, 12.65 mm from the magnet housing surface. Plastic spacers were used to create the change in height because they do not interfere with the magnetic field. In this experiment three replicate measurements were made for each of the 48 conditions giving a total of 144 measurements. The results from this experiment are shown in the chart of Figure 7.

The magnetic field characteristics for the various magnets tested are shown in Table 2:

Table 2 - Magnetic Field Characteristics for Magnet Embodiments

| Magnet | a (G) | α STDEV(G) | β (1/mm) | β STDEV (1/mm) | R2 |
|---|---|---|---|---|---|
| BLUE | 4537 | 17.60 | 0.324 | 0.0034 | 0.9994 |
| NS | 3588 | 9.21 | 0.436 | 0.0030 | 0.9998 |
| SH | 4086 | 13.58 | 0.463 | 0.0041 | 0.9997 |
| LH | 4878 | 15.94 | 0.228 | 0.0021 | 0.9994 |
| HH H Pole | 5846 | 8.15 | 0.266 | 0.0010 | 0.9999 |
| HH N Pole | 2996 | 24.14 | 0.551 | 0.0117 | 0.9974 |

The magnetic field strength at the surface in decreasing order were HH H Pole, LH, DEXTER DESIGN, SH, NS and HH N Pole, which had magnitudes of 5800 G (0.58 T), 4900 G (0.49 T), 4500 G (0.45 T), 4100 G (0.41 T), 3600 G (0.36 T), and 3000 (0.3 T), respectively. The magnetic field decay rates in decreasing order belong to the HH N Pole, SH, NS, DEXTER DESIGN, HH H Pole, and LH, which had values of 0.55 mm$^{-1}$, 0.46 mm$^{-1}$, 0.44 mm$^{-1}$, 0.32 mm$^{-1}$, 0.27 mm$^{-1}$, and 0.23 mm$^{-1}$, respectively. A larger magnetic field decay rate corresponds to a steeper field gradient.

The HH did not have a uniform field; the HH H Pole had the largest field magnitude whereas the HH N Pole had the weakest. By using the same amount of magnetic material and only changing the magnet orientation, the HH H Pole generated a 61% larger field compared to the NS. The magnetic field decay rate for the HH H Pole was 39% smaller than the NS decay rate, while the HH N Pole was 25% larger than the NS decay rate.

The LH had the second largest field magnitude and a uniform field over all poles (other locations were not tested). The magnetic field magnitude was 36% larger than the NS field magnitude and the magnetic field decay rate was 48% smaller than the NS rate. The LH's strong initial magnetic field and shallow field gradient allows for separation of large height ranges.

The SH had a uniform field over the poles. The magnetic field magnitude was only slightly larger (14%) than the NS field magnitude, and the magnetic field rate was slightly larger (5%) than the NS rate. The AVG MFD for this magnet is expected to be small because it has a small initial magnetic field and shallow gradient.

Both the LH and HH H Pole magnets had larger initial magnetic fields and smaller field decay rates compared to the DEXTER DESIGN magnet. This means the prototypes of the LH embodiment and HH configuration generated a larger field and shallower gradient using less magnetic material and weaker magnets as compared to the Dexter design. Therefore, these configurations may provide a much larger AVG MFD, in turn making the processing of much larger separation height ranges (larger volumes) possible.

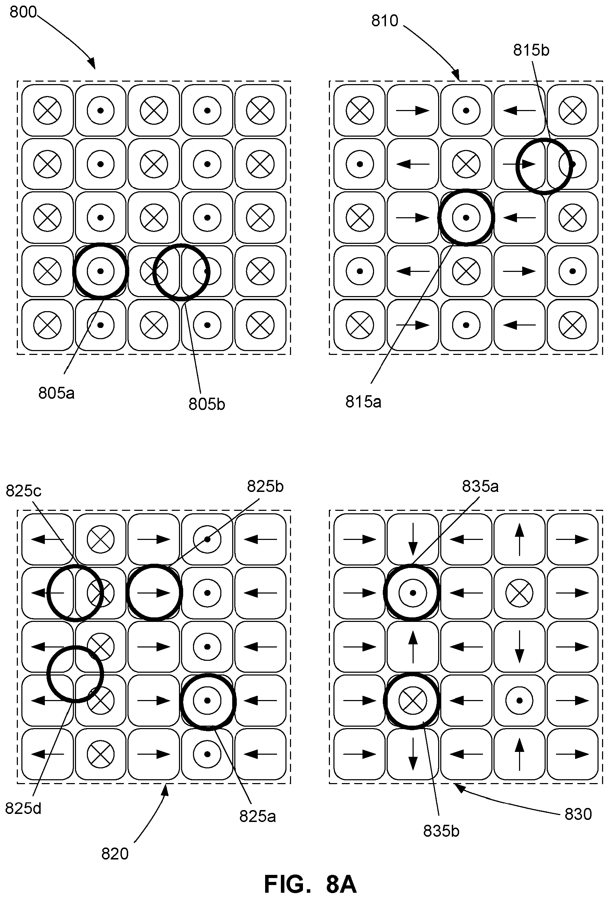

Consistent separation performance is the driving force for a plate magnet having a uniform field. In theory, if the magnetic field is uniform, the separation performance should be consistent. Uniformity was tested for each alternative magnet configuration as well as the control magnets by performing CD3 positive cell selection separations at different locations on each magnet. If the recovery of CD3 cells was consistent from location to location the magnet was considered uniform (at least for cell separation purposes). As shown in Figure 8A, the NS configuration and Dexter Design magnets 800 was tested at positions 805a and 805b. The SH configuration 810 was tested at positions 815a and 815b. The example LH embodiment 820 was tested at positions 825a-825d. The HH configuration 830 was tested at positions 835a and 835b.

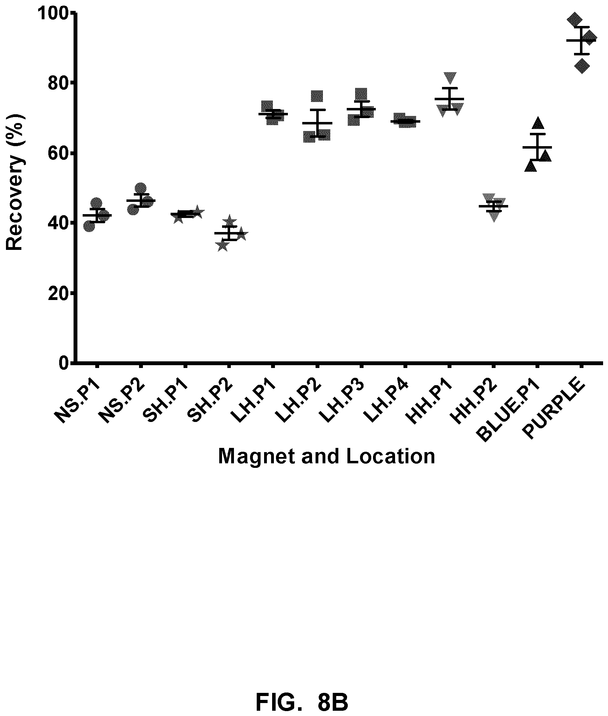

The PURPLE magnet (available from Stemcell Technologies) was used as control for maximum recovery. The Dexter and NS magnets have four independent (non-repeating locations), SH and LH have six independent locations, and the HH has 12 independent locations. For the plate magnets, 2 mL vials were used as they could be easily placed directly over a desired location. The process volume used was a 0.5 mL capture volume and 0.1 mL was left behind. This means, theoretically, the baseline recovery should be 20%. The negative fraction was removed using aspiration with micropipette. The PURPLE magnet used a 5 mL tube and had a process volume of 2 mL. In this experiment, there were 12 conditions with three replicates, for a total of 36 separations. The separations were run in a random block design. The results from this experiment can be seen in Figure 8B.

The NS, SH, and LH were found to have consistent recoveries, indicating they have uniform fields. The HH, as expected, did not have consistent recoveries between the HH H Pole (P1), and HH N Pole (P2); indicating its magnetic field was not uniform. For a 10 minute separation, the average LH recovery was 70.21%, which was 59% more than the NS recovery of 44.28%. The average HH H Pole recovery was 75.35%, which 70% more than the NS recovery. These recoveries are larger than the average Dexter magnet design with a recovery of 61.64%. The SH and the HH N Pole had average recoveries of 39.26% and 44.76%, respectively, which were both lower than the average NS recovery. The SH recoveries were lower than the NS most likely because the field magnitude is the same, but the gradient is slightly shallower.

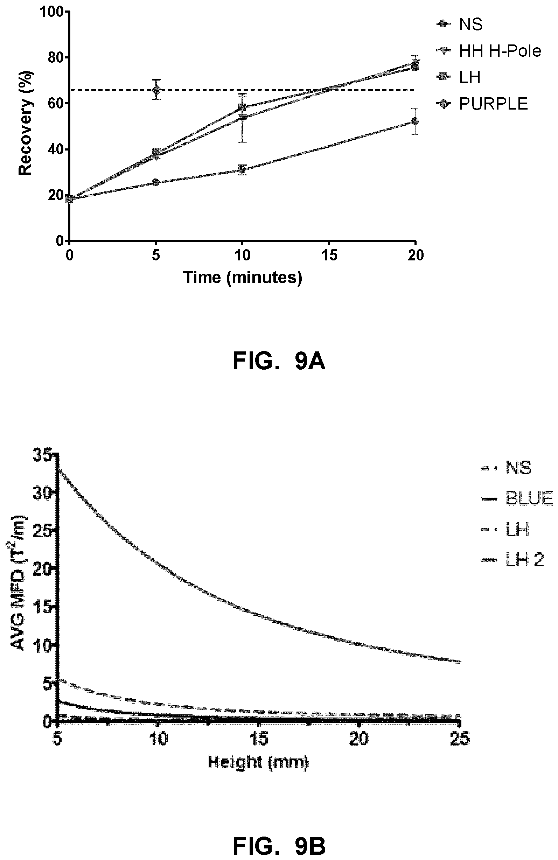

Next, the recovery as a function of time (recovery rate) was characterized for each prototype of the alternative magnet embodiments. This may provide a better understanding of how recovery varies with time for each plate magnet system. For this experiment, the separation was a CD3 positive cell selection, with a 0.5 mL process volume, and 0.1 mL capture volume. Again, the theoretical baseline recovery is 20%. The maximum recovery was found by completing a 2 mL PURPLE magnet separation. The different plate magnets used were the NS, LH, and HH, and all separations were done on the P1 location (from last experiment). Each magnet had separations completed at time intervals of 0, 5, 10, and 20 minutes. Again, 2 mL vials were used as they could be easily placed directly over the desired location. In this experiment, there were 11 conditions with two replicates (three for PURPLE), for a total of 23 separations. The separations were run in a random block design. The recovery and change in purity odds ratio versus time results from these experiments can be seen in Figures 9A and 9B.

The LH and HH H Pole had similar recovery rates. This is because the LH and HH H Pole had similar AVG MFD over the separation height range for the current experimental setup. The 2 mL vial sits 5 mm above the magnet surface; therefore the HH H Pole with its slightly steeper field gradient loses the advantage of its higher initial magnetic field. The recovery rates for the LH and HH H Pole are about twice the recovery rate of the NS.

Next, the LH embodiment and Dexter design magnets were compared head to head, even though the Dexter plate magnet had more magnetic material and higher grade magnets. This experiment gave a baseline of how the prototype LH compared to the Dexter design. This information was used to assess the potential of a second Linear Halbach (LH2) prototype with larger and higher grade magnets.

The recovery rates of the LH and Dexter magnets were compared head to head, and the cell sedimentation rate was quantified. The sedimentation of non-target cells can lead to a decrease in final purity. For this experiment, the separation was a CD3 positive cell selection, with a 1 mL process volume, and 0.1 mL capture volume. Therefore, the baseline recovery is 10%. The maximum recovery was found by completing a 2 mL PURPLE magnet separation. The different plate magnets used were the LH and the Dexter design, and all separations were done on the P1 location (described above). Each magnet had separations completed at time intervals of 0, 3, 5, 8, 10, 15, 25, 40 and 60 minutes.

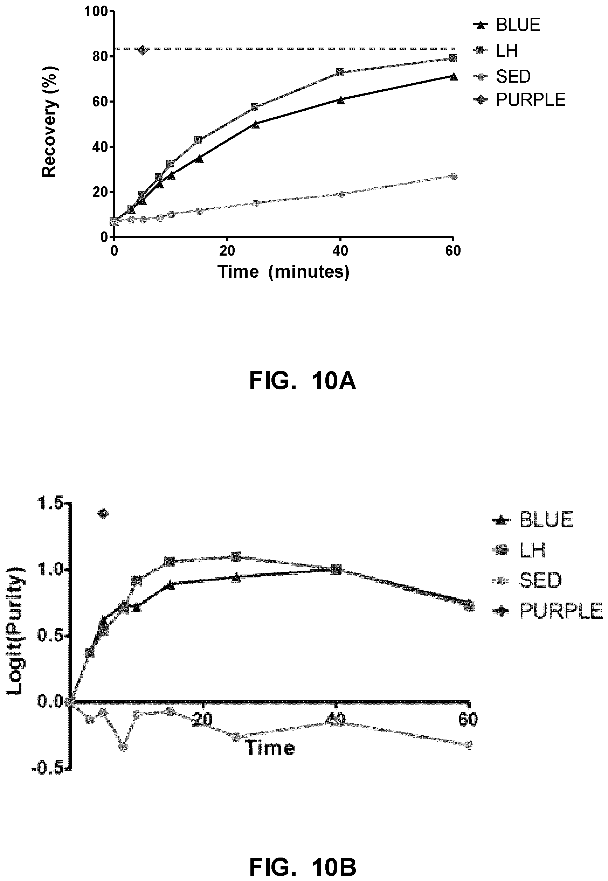

A sedimentation condition (SED) that was not placed on a magnet was also processed at the same time intervals. Again, 2 mL vials were used as they could be easily placed directly over the desired location. In this experiment, there were 26 conditions with two replicates, for a total of 52 separations. Since there were so many separations the two replicates for each condition were mixed prior to flow cytometry analysis. This provides a manual average of the replicates. The separations were run in a random block design. The recovery and change in purity odds ratio versus time results from this experiment can be seen in Figures 10A (recovery versus time for LH, BLUE, and SED) and 10B (purity change in odds ratio versus time for LH, BLUE and SED).

The prototype LH embodiments used 1/4", N42 grade, rare earth, cube magnets as the magnetic elements. A further embodiment of the LH design (referred to as LH2) may employ 1/2", N52 grade, rare earth, cube magnets compared to the 1/4", N42 grade, rare earth, cube magnets as the magnetic elements. The AVG MFD was estimated for the LH2 embodiment over a range of process volume heights and a constant capture volume height. The AVG MFD was also simulated for the NS, Dexter, and LH for contrast. Figure 11 shows the AVG MFD as a function of process volume height for the NS configuration, Dexter configuration, and the LH and LH2 embodiments. The capture volume height has been set to 4.99 mm and the process volume height varies from 5 mm to 25 mm.

As Figure 11 illustrates, the LH2 embodiment is expected to produce a much stronger average magnetic force on the cells compared to the DEXTER DESIGN magnet because of its larger AVG MFD. This suggests that the LH2 design will be much stronger than the current Dexter design, therefore it should have the capabilities to process a large separation height range (potentially up to an inch). Depending on the hardware used, the LH2 should be able to process small to extra-large process volumes.

The linear Halbach array is not a stable configuration of the magnet elements. As a result, a suitable housing may be required to hold the elements of the linear Halbach array in place. Stronger magnetic elements may cause large internal stresses in the housing; therefore a strong material may be required for the housing. Aluminum is one example of a suitable housing material because it has a large strength to weight ratio, and is a non-magnetic material. By contrast, while steel is very strong, it is ferromagnetic and may disturb the magnetic field.

In some cases, set screws may be used to secure magnetic elements in place. Outer posts, e.g. guide elements such as four outer posts extending about a 1/2" from the surface of the housing may be used as a safety precaution. These posts may stop the magnet from getting too close to large ferromagnetic objects. This may also protect users from getting their hands crushed. The posts can be used to protect users from large ferromagnetic objects. Small ferromagnetic objects may still have access to the magnet surface, so users should handle the magnet with care. The thickness of the top plate/cover may be minimized in order to maximize the AVG MFD.

To achieve maximum uniformity over a finite surface plane the size and shape of the magnetic elements in the linear Halbach array can be varied and tuned dependent on the overall size of the array. For example, in an array of 85 mm x 130 mm (standard ANSI plate dimensions) alternating blocks of small cubic magnet elements can be assembled with larger (double size) blocks to reduce the boundary effects of a finite linear Halbach array (see e.g. Figure 15). Designs can be created that, while sacrificing magnetic strength, result in an overall more uniform magnetic field across the surface of the array. Thus, Linear Halbach Arrays can be optimized to create both a very strong magnetic field gradient and a uniform field that enables, for example, cell separation to be carried out reproducibly across the magnetic surface independent of the precise positioning of the sample on the surface of the magnet array.

As array elements scale relative to the size of the array inhomogeneity arises. In such cases, variable sized magnetic elements may be used in the array to improve field uniformity.

While the above description describes features of example embodiments, it will be appreciated that some features and/or functions of the described embodiments are susceptible to modification without departing from the principles of operation of the described embodiments. For example, the various characteristics which are described by means of the represented embodiments or examples may be selectively combined with each other. Accordingly, what has been described above is intended to be illustrative of the claimed concept and non-limiting. It will be understood by persons skilled in the art that other variants and modifications may be made without departing from the scope of the invention as defined in the claims appended hereto. The scope of the claims should not be limited by the preferred embodiments and examples, but should be given the broadest interpretation consistent with the description as a whole.

**Claims**

1. A magnetic separation apparatus comprising:

   a) a plurality of magnetic elements defining a first two-dimensional magnetic array including a first dimension and a second dimension; and
   b) a housing supporting the plurality of magnetic elements;

   wherein

   c) the two-dimensional magnetic array includes a plurality of one-dimensional magnetic arrays, each one-dimensional magnetic array comprising a plurality of the magnetic elements positioned adjacent one another and rotated 90 degrees about the same axis with respect to each adjacent magnetic element in the same one-dimensional magnetic array, extending in a first direction along the first dimension, and oriented to define a Halbach array, the plurality of one dimensional arrays being positioned adjacent to one another and extending in a second direction along the second dimension;
   d) the plurality of magnetic elements define an array surface having a magnetic field configured to attract magnetically susceptible particles towards the array surface; and
   e) the magnetic fields of the plurality of one-dimensional arrays are oriented in the same direction along the array surface.

2. The magnetic separation apparatus of claim 1, wherein the array surface is substantially planar, and optionally substantially rectangular.

3. The magnetic separation apparatus of claim 1 or 2, wherein the housing defines a container receiving surface adjacent to the array surface and/or a container receiving area shaped to receive at least one container configured to contain the plurality of magnetically susceptible particles.

4. The magnetic separation apparatus of claim 3, further comprising a plurality of guide elements, the guide elements arranged to direct one or more containers to a position adjacent the container receiving surface, and optionally the guide elements include at least one guide member extending from the housing in a direction perpendicular to the container receiving surface and away from the array surface.

5. The magnetic separation apparatus of any one of claims 1 to 4, further comprising a second plurality of magnetic elements arranged to define a second two-dimensional magnetic array.

6. The magnetic separation apparatus claim 5, wherein:

   a) the first two-dimensional magnetic array and the second two-dimensional magnetic array are positioned in an at least partially facing arrangement with the array surface of the first two-dimensional magnetic array facing a second array surface of the second two-dimensional magnetic array; and
   b) the apparatus comprises a receiving area between the first two-dimensional magnetic array and the second two-dimensional magnetic array shaped to receive a container for a plurality of magnetically susceptible particles.

7. The magnetic separation apparatus of claim 6, wherein the first two-dimensional magnetic array and the second two-dimensional magnetic array are substantially parallel.

8. The magnetic separation apparatus of claim 6 or 7, wherein the first two-dimensional magnetic array and the second two-dimensional magnetic array are oriented to define a magnetic field strength minimum along a plane defined by the magnetic field strength minimum resulting from the magnetic field of the first two-dimensional magnetic array and the magnetic field of the second two-dimensional magnetic array.

9. The magnetic separation apparatus of claim 5, wherein the first two-dimensional magnetic array and the second two-dimensional magnetic array are arranged:

   a) back to back; or
   b) to provide a substantially planar array with the first and second two-dimensional arrays positioned adjacent to one another to provide a larger array.

10. The magnetic separation apparatus of any one of claims 1 to 9, wherein the magnetic elements comprise permanent magnets and/or electromagnets.

11. A method for magnetically separating a plurality of targets from a suspension in a container unit, the method comprises:

a) linking the plurality of targets with magnetically susceptible particles to provide a plurality of linked particles in the suspension;
b) positioning the container unit in proximity to an array surface of a two dimensional magnet array, the two-dimensional magnet array comprising a plurality of magnetic elements arranged to define a plurality of one dimensional Halbach arrays that each extend in a first dimension, the plurality of magnetic elements of each one dimensional Halbach array are rotated 90 degrees about the same axis with respect to each adjacent magnetic element in the same one-dimensional array, the plurality of one-dimensional Halbach arrays positioned adjacent to one another extending in a second dimension and, wherein the magnetic fields defined by the plurality of one-dimensional Halbach arrays are oriented in the same direction along the array surface; and
c) removing a portion of the suspension from the container unit while the container unit is positioned in proximity to the array surface.

12. The method of claim 11, wherein positioning the container unit in proximity to the array surface comprises guiding the container unit towards the array surface using a plurality of guide elements.

13. The method of claim 12, wherein the plurality of guide elements are arranged to correspond to a shape of the container unit, and optionally the container unit is one of a tube, a vial, a Petri dish, a bottle, a bag, and a multi-well micro-plate.

14. The method of any one of claims 11 to 13, wherein:

a) the container unit comprises a plurality of container compartments, each container compartment shaped to receive an individual container, and at least one of the container compartments comprises an in-use container with a corresponding suspension; and
b) the method comprises repeating the linking and extracting steps for the corresponding suspension of each in-use container.

**Patentansprüche**

1. Magnetische Trennvorrichtung, umfassend:

a) eine Vielzahl von magnetischen Elementen, die ein erstes zweidimensionales magnetisches Array definieren, das eine erste Dimension und eine zweite Dimension beinhaltet; und
b) ein Gehäuse, das die Vielzahl von magnetischen Elementen trägt; wobei
c) das zweidimensionale magnetische Array eine Vielzahl von eindimensionalen magnetischen Arrays beinhaltet, wobei jedes eindimensionale magnetische Array eine Vielzahl der magnetischen Elemente umfasst, die einander benachbart positioniert und um 90 Grad um dieselbe Achse in Bezug auf jedes benachbarte magnetische Element in demselbem eindimensionalen magnetischen Array gedreht sind, sich in einer ersten Richtung entlang der ersten Dimension erstrecken und so ausgerichtet sind, dass sie ein Halbach-Array definieren, wobei die Vielzahl von eindimensionalen Arrays zueinander benachbart positioniert sind und sich in einer zweiten Richtung entlang der zweiten Dimension erstrecken;
d) die Vielzahl von magnetischen Elementen eine Array-Oberfläche mit einem Magnetfeld definieren, das so konfiguriert ist, dass es magnetisch empfindliche Partikel in Richtung der Array-Oberfläche anzieht; und
e) die Magnetfelder der Vielzahl von eindimensionalen Arrays in der gleichen Richtung entlang der Array-Oberfläche orientiert sind.

2. Magnetische Trennvorrichtung nach Anspruch 1, wobei die Array-Oberfläche im Wesentlichen eben und optional im Wesentlichen rechteckig ist.

3. Magnetische Trennvorrichtung nach Anspruch 1 oder 2, wobei das Gehäuse eine Behälteraufnahmefläche benachbart zu der Array-Oberfläche und/oder einen Behälteraufnahmebereich definiert, der so geformt ist, dass er mindestens einen Behälter aufnimmt, der so konfiguriert ist, dass er die Vielzahl von magnetisch empfindlichen Partikeln enthält.

4. Magnetische Trennvorrichtung nach Anspruch 3, ferner umfassend eine Vielzahl von Führungselementen, wobei die Führungselemente so angeordnet sind, dass sie einen oder mehrere Behälter zu einer Position neben der Behälteraufnahmefläche führen, und optional die Führungselemente mindestens ein Führungsglied beinhalten, das sich von dem Gehäuse in einer Richtung senkrecht zur Behälteraufnahmefläche und weg von der Array-Oberfläche erstreckt.

5. Magnetische Trennvorrichtung nach einem der Ansprüche 1 bis 4, ferner umfassend eine zweite Vielzahl von magnetischen Elementen, die so angeordnet sind, dass sie ein zweites zweidimensionales magnetisches Array definieren.

6. Magnetische Trennvorrichtung nach Anspruch 5, wobei:

   a) das erste zweidimensionale magnetische Array und das zweite zweidimensionale magnetische Array in einer zumindest teilweise gegenüberliegenden Anordnung positioniert sind, wobei die Array-Oberfläche des ersten zweidimensionalen magnetischen Arrays einer zweiten Array-Oberfläche des zweiten zweidimensionalen magnetischen Arrays zugewandt ist; und

   b) die Vorrichtung einen Aufnahmebereich zwischen dem ersten zweidimensionalen magnetischen Array und dem zweiten zweidimensionalen magnetischen Array umfasst, der so geformt ist, dass er einen Behälter für eine Vielzahl von magnetisch empfindlichen Partikeln aufnimmt.

7. Magnetische Trennvorrichtung nach Anspruch 6, wobei das erste zweidimensionale magnetische Array und das zweite zweidimensionale magnetische Array im Wesentlichen parallel sind.

8. Magnetische Trennvorrichtung nach Anspruch 6 oder 7, wobei das erste zweidimensionale magnetische Array und das zweite zweidimensionale magnetische Array so ausgerichtet sind, dass sie ein Magnetfeldstärkeminimum entlang einer Ebene definieren, die durch das Magnetfeldstärkeminimum definiert ist, das sich aus dem Magnetfeld des ersten zweidimensionalen magnetischen Arrays und dem Magnetfeld des zweiten zweidimensionalen magnetischen Arrays ergibt.

9. Magnetische Trennvorrichtung nach Anspruch 5, wobei das erste zweidimensionale magnetische Array und das zweite zweidimensionale magnetische Array angeordnet sind:

   a) Rücken an Rücken; oder

   b) um ein im Wesentlichen ebenes Array bereitzustellen, wobei das erste und das zweite zweidimensionale Array benachbart zueinander positioniert sind, um ein größeres Array bereitzustellen.

10. Magnetische Trennvorrichtung nach einem der Ansprüche 1 bis 9, wobei die magnetischen Elemente Permanentmagnete und/oder Elektromagnete umfassen.

11. Verfahren zum magnetischen Trennen einer Vielzahl von Targets aus einer Suspension in einer Behältereinheit, wobei das Verfahren umfasst:

   a) Verknüpfen der Vielzahl von Targets mit magnetisch empfindlichen Partikeln, um eine Vielzahl von verknüpften Partikeln in der Suspension bereitzustellen;

   b) Positionieren der Behältereinheit in der Nähe einer Array-Oberfläche eines zweidimensionalen magnetischen Arrays, wobei das zweidimensionale magnetische Array eine Vielzahl von magnetischen Elementen umfasst, die so angeordnet sind, dass sie eine Vielzahl von eindimensionalen Halbach-Arrays definieren, die sich jeweils in einer ersten Dimension erstrecken, wobei die Vielzahl von magnetischen Elementen jedes eindimensionalen Halbach-Arrays um 90 Grad um dieselbe Achse in Bezug auf jedes benachbarte magnetische Element in demselben eindimensionalen Array gedreht werden, wobei sich die Vielzahl von zueinander benachbart positionierten eindimensionalen Halbach-Arrays in einer zweiten Dimension erstrecken und wobei die Magnetfelder, die durch die Vielzahl von eindimensionalen Halbach-Arrays definiert sind, in der gleichen Richtung entlang der Array-Oberfläche orientiert sind; und

   c) Entfernen eines Teils der Suspension aus der Behältereinheit, während die Behältereinheit in der Nähe der Array-Oberfläche positioniert ist.

**12.** Verfahren nach Anspruch 11, wobei das Positionieren der Behältereinheit in der Nähe der Array-Oberfläche das Führen der Behältereinheit in Richtung der Array-Oberfläche unter Verwendung einer Vielzahl von Führungselementen umfasst.

**13.** Verfahren nach Anspruch 12, wobei die Vielzahl von Führungselementen so angeordnet sind, dass sie einer Form der Behältereinheit entsprechen, und optional die Behältereinheit eines von einem Röhrchen, einem Fläschchen, einer Petrischale, einer Flasche, einem Beutel und einer Mikrotiterplatte ist.

**14.** Verfahren nach einem der Ansprüche 11 bis 13, wobei:

a) die Behältereinheit eine Vielzahl von Behälterfächern umfasst, wobei jedes Behälterfach so geformt ist, dass es einen einzelnen Behälter aufnimmt, und mindestens

eines der Behälterfächer einen in Gebrauch befindlichen Behälter mit einer entsprechenden Suspension umfasst; und
b) das Verfahren das Wiederholen der Verknüpfungs- und Extraktionsschritte für die entsprechende Suspension jedes in Gebrauch befindlichen Behälters umfasst.


**Revendications**

**1.** Appareil de séparation magnétique comprenant :

a) une pluralité d'éléments magnétiques définissant un premier réseau magnétique à deux dimensions comprenant une première dimension et une seconde dimension ; et
b) un boîtier supportant la pluralité d'éléments magnétiques ;
c) ledit réseau magnétique à deux dimensions comprenant une pluralité de réseaux magnétiques à une dimension, chaque réseau magnétique à une dimension comprenant une pluralité d'éléments magnétiques positionnés les uns à côté des autres et tournés de 90 degrés autour du même axe par rapport à chaque élément magnétique adjacent dans le même réseau magnétique à une dimension, s'étendant dans une première direction le long de la première dimension, et orienté pour définir un réseau de Halbach, la pluralité de réseaux à une dimension étant positionnés adjacents les uns aux autres et s'étendant dans une seconde direction le long de la seconde dimension ;
d) ladite pluralité d'éléments magnétiques définissant une surface de réseau possédant un champ magnétique conçu pour attirer des particules magnétiquement sensibles vers la surface de réseau ; et
e) lesdits champs magnétiques de la pluralité de réseaux à une dimension étant orientés dans la même direction le long de la surface de réseau.

**2.** Appareil de séparation magnétique selon la revendication 1, ladite surface du réseau étant sensiblement plane et éventuellement sensiblement rectangulaire.

**3.** Appareil de séparation magnétique selon la revendication 1 ou 2, ledit boîtier définissant une surface de réception de contenant adjacente à la surface de réseau et/ou une zone de réception de contenant façonnée pour recevoir au moins un contenant conçu pour contenir la pluralité de particules magnétiquement sensibles.

**4.** Appareil de séparation magnétique selon la revendication 3, comprenant en outre une pluralité d'éléments guides, les éléments guides agencés pour diriger un ou plusieurs contenants vers une position adjacente à la surface de réception de contenant, et éventuellement les éléments guides comprennent au moins un élément de guidage s'étendant à partir du boîtier dans une direction perpendiculaire à la surface de réception du contenant et en s'éloignant de la surface du réseau.

**5.** Appareil de séparation magnétique selon l'une quelconque des revendications 1 à 4, comprenant en outre une seconde pluralité d'éléments magnétiques agencés pour définir un second réseau magnétique à deux dimensions.

**6.** Appareil de séparation magnétique selon la revendication 5 :

a) ledit premier réseau magnétique à deux dimensions et ledit second réseau magnétique à deux dimensions étant positionnés dans un agencement au moins partiellement en vis-à-vis avec la surface de réseau du premier réseau magnétique à deux dimensions faisant face à une seconde surface de réseau du second réseau magnétique à deux dimensions ; et

b) ledit appareil comprenant une zone de réception entre le premier réseau magnétique à deux dimensions et le second réseau magnétique à deux dimensions façonnée pour recevoir un contenant pour une pluralité de particules magnétiquement sensibles.

7.  Appareil de séparation magnétique selon la revendication 6, ledit premier réseau magnétique à deux dimensions et ledit second réseau magnétique à deux dimensions étant sensiblement parallèles.

8.  Appareil de séparation magnétique selon la revendication 6 ou 7, ledit premier réseau magnétique à deux dimensions et ledit second réseau magnétique à deux dimensions étant orientés pour définir un minimum d'intensité de champ magnétique le long d'un plan défini par le minimum d'intensité de champ magnétique résultant du champ magnétique du premier réseau magnétique à deux dimensions et du champ magnétique du second réseau magnétique à deux dimensions.

9.  Appareil de séparation magnétique selon la revendication 5, ledit premier réseau magnétique à deux dimensions et ledit second réseau magnétique à deux dimensions étant agencés :

   a) dos à dos ; ou
   b) pour fournir un réseau sensiblement plan avec les premier et second réseaux à deux dimensions positionnés adjacents l'un à l'autre pour fournir un réseau plus grand.

10. Appareil de séparation magnétique selon l'une quelconque des revendications 1 à 9, lesdits éléments magnétiques comprenant des aimants permanents et/ou des électroaimants.

11. Procédé permettant la séparation de manière magnétique d'une pluralité de cibles d'une suspension dans une unité de contenant, le procédé comprend :

   a) la liaison de la pluralité de cibles avec des particules magnétiquement sensibles pour fournir une pluralité de particules liées dans la suspension ;
   b) le positionnement de l'unité de contenant à proximité d'une surface de réseau d'un réseau d'aimants à deux dimensions, le réseau d'aimants à deux dimensions comprenant une pluralité d'éléments magnétiques agencés pour définir une pluralité de réseaux de Halbach à une dimension qui s'étendent chacun dans une première dimension, la pluralité d'éléments magnétiques de chaque réseau de Halbach à une dimension sont tournés de 90 degrés autour du même axe par rapport à chaque élément magnétique adjacent dans le même réseau à une dimension, la pluralité de réseaux de Halbach à une dimension positionnés adjacents les uns aux autres s'étendant dans une seconde dimension et, lesdits champs magnétiques définis par la pluralité de réseaux de Halbach à une dimension étant orientés dans la même direction le long de la surface de réseau ; et
   c) le retrait d'une partie de la suspension de l'unité de contenant pendant que l'unité de contenant est positionnée à proximité de la surface de réseau.

12. Procédé selon la revendication 11, ledit positionnement de l'unité de contenant à proximité de la surface de réseau comprenant le guidage de l'unité de contenant vers la surface de réseau à l'aide d'une pluralité d'éléments guides.

13. Procédé selon la revendication 12, ladite pluralité d'éléments guides étant agencés pour correspondre à une forme de l'unité de contenant, et éventuellement ladite unité de contenant étant l'un d'un tube, d'un flacon, d'une boîte de Pétri, d'une bouteille, d'un sac et d'une microplaque multipuits.

14. Procédé selon l'une quelconque des revendications 11 à 13,

   a) ladite unité de contenant comprenant une pluralité de compartiments à contenant, chaque compartiment à contenant étant façonné pour recevoir un contenant individuel, et au moins l'un des compartiments à contenant comprenant un contenant en cours d'utilisation avec une suspension correspondante ; et
   b) ledit procédé comprenant la répétition des étapes de liaison et d'extraction pour la suspension correspondante de chaque contenant en cours d'utilisation.

① Add EasySep™ Isolation Cocktail to single cell suspension

Incubate 5 minutes†

② Add EasySep™ RapidSpheres™ to cell suspension

No Incubation*

③ Immediately place tube in EasySep™ Magnet and incubate 3 minutes†

④ Pour off desired fraction into a new tube

FIG. 1

FIG. 2

FIG. 3A

FIG. 3B

FIG. 3C

FIG. 3D

Single Magnet
(3/16" Cube)

Alternating
Magnets

Super
Halbach

Single
Halbach

Hyper
Halbach

A = ~.65 in.

A=Single Magnet k-Gauss
(3.84 KG @ Surface)

B=Single Halbach k-Gauss

C=Hyper Halbach k-Gauss

K-Gauss

Air Gap

**FIG. 4**

EP 3 452 590 B1

FIG. 5A

FIG. 5B

FIG. 6A

FIG. 6B

Curve Fit: $y = \alpha e^{-\beta x}$

Legend:
- Blue
- NS
- SH
- LH
- HH H Pole
- HH N Pole

FIG. 7

**FIG. 8A**

FIG. 8B

FIG. 9A

FIG. 9B

FIG. 10A

FIG. 10B

FIG. 11

**FIG. 12**

FIG. 13

FIG. 14

FIG. 15

**FIG. 16**

FIG. 17A

FIG. 17B

**REFERENCES CITED IN THE DESCRIPTION**

**Patent documents cited in the description**

- US 6514415 B2
- WO 2008080047 A
- WO 9942219 A
- WO 0171034 A

**Non-patent literature cited in the description**

- **LESTER BARNSLEY et al.** Halbach arrays consisting of cubic elements optimized for high field gradients in magnetic drug targeting applications. *PHYSICS IN MEDICINE AND BIOLOGY,* 12 October 2015, vol. 60 (21), 8303-8327
- **M. ZBOROWSKI.** Magnetic Cell Separation. Elsevier, 2008